# EUROPEAN PATENT SPECIFICATION

(11) **EP 2 393 831 B1**
(45) Date of publication and mention of the grant of the patent: **18.05.2016**
(21) Application number: 10703194.0
(22) Date of filing: 04.02.2010
(51) Int. Cl.: C07K 14/435, A61K 38/00

(54) **MODIFIED OMCI AS A COMPLEMENT INHIBITOR**
MODIFIZIERTES OMCI ALS KOMPLEMENTINHIBITOR
POLYPEPTIDE OMCI MODIFIÉ UTILISÉ EN TANT QU'INHIBITEUR DE COMPLÉMENT

(30) Priority: 05.02.2009 WO PCT/GB2009/000311; 20.04.2009 GB 0906779
(43) Date of publication of application: 14.12.2011
(73) Proprietor: Volution Immuno Pharmaceuticals SA, 3461-1211 Geneve 3 (CH)
(72) Inventor: NUNN, Miles, A., Wallingford OX10 8BB (GB); LEA, Susan, M., Oxford OX1 3RE (GB); ROVERSI, Pietro, C., Oxford OX1 3RE (GB)
(74) Representative: Carpmaels & Ransford LLP
(86) International application number: PCT/GB2010/000213
(87) International publication number: WO 2010/100396

(56) References cited:
- WO-A2-2004/106369
- WO-A2-2009/098454
- ROVERSI ET AL: "The Structure of OMCI, a Novel Lipocalin Inhibitor of the Complement System" JOURNAL OF MOLECULAR BIOLOGY, LONDON, GB LNKD- DOI:10.1016/J.JMB.2007.03.064, vol. 369, no. 3, 11 May 2007 (2007-05-11), pages 784-793, XP022077570 ISSN: 0022-2836
- HEPBURN N J ET AL: "In vivo characterization and therapeutic efficacy of a C5-specific inhibitor from the soft tick Ornithodoros moubata" JOURNAL OF BIOLOGICAL CHEMISTRY, AMERICAN SOCIETY FOR BIOCHEMISTRY AND MOLECULAR BIOLOGY, INC, US LNKD- DOI:10.1074/JBC.M609858200, vol. 282, no. 11, 16 March 2007 (2007-03-16), pages 8292-8299, XP002564815 ISSN: 0021-9258 [retrieved on 2007-01-10]

## Description

### Field of the invention

The present invention relates to novel compositions useful in the treatment of disease and conditions mediated by complement and in particular to modified tick-derived specific inhibitors of complement and their use for treatment of diseases and conditions mediated by complement.

### Background of the invention

Complement is an important innate immune defence system. It is involved in the protection of the body from foreign agents and in the process of inflammation. There are over 30 serum and cell surface proteins that are known to be involved in the function and regulation of the complement system.

There are three pathways of complement activation: the classical pathway; the alternative pathway and the lectin pathway. The classical pathway is activated by IgM or IgG complexes or carbohydrates. The alternative pathway is activated by non-self surfaces and bacterial endotoxins. The lectin pathway is activated by mannan-binding lectin (MBL) binding to mannose on the surface of a pathogen. The three pathways of complement comprise parallel cascades in which similar forms of the C3-convertase and a C5-convertase cleave and activate components C3 and C5 respectively, creating C3a and C3b and C5a and C5b.

These active complement fragments are responsible for mediating a wide range of immune effects. C3a and C5a trigger degranulation of mast cells and increase vascular permeability and smooth muscle contraction. C5a also acts as a chemotactic protein and recruits immune cells. C3b opsonises and increases the phagocytosis of pathogens. C5b is responsible for initiating membrane attack complex (MAC) formation.

The activation of the three complement pathways is carefully regulated under physiological conditions in healthy individuals. Due to the important role of complement in the immune system and the consequences of inappropriate complement activation, there are multiple mechanisms which act together to regulate complement pathway activation. The main regulators of the complement pathways are complement control proteins. These are expressed in the plasma at higher concentrations than the complement components themselves. Some complement control proteins are expressed on the surface of the body's own cells, thus preventing these cells from being inappropriately targeted by complement. Additionally, many of the active complement components, such as C3a and C5a, have very short half-lives and hence are only active in the plasma for short periods after their activation.

Failure of the control mechanisms which regulate complement activation can result in damage to the body's own tissues. Failure of the complement control mechanisms has been implicated in many pathological conditions and diseases. Conditions known to involve a lack of control of the complement pathways include age-related macular degeneration (AMD), Alzheimer's disease, allergic encephalomyelitis, allotransplatation, arthritis of various sorts including rheumatoid arthritis, asthma, adult respiratory distress syndrome, burn injuries, cancer, Crohn's disease, dermatomyositis, glomerulonephritis, haemolytic anaemia, haemodialysis, hereditary angioedema, idiopathic membranous nephropathy, *in utero* growth restriction (IUGR), ischaemia reperfusion injuries, motor neuron disease, multiple system organ failure, multiple sclerosis, myasthenia gravis, myocardial infarction, nephritis, pemphigoid, post cardiopulmonary bypass, psoriasis, septic shock, spontaneous miscarriage, stroke, systemic lupus erythematosus, uveitis, vascular leak syndrome and xenotransplantation.

Due to the importance of the careful regulation of the complement pathways, there has been much interest in the development of complement inhibitors for use in the prevention or treatment or conditions and diseases known to involve a failure to regulate complement. Research has focused on the development of antibodies for specific complement components, RNA aptamers and molecules that target complement receptors.

Eicosanoids are a family of oxygenated biologically active lipid mediators that are derived from the 20-carbon fatty acid arachidonate (AA) and induce numerous effects on diverse cell types and organs. The leukotrienes (LKs) are a subfamily of eicosanoids that have multiple effects, including regulation of vascular tone and permeability of capillaries and venules, contraction or relaxation of muscle (cysteinyl LKs), control of growth and or spread of malignant cells (Schwartz *et al.,* 2005; Aya, 2006), and activation of leukocytes, in particular in autoimmune and inflammatory conditions (LKs, HETEs) (Samuelsson, 1983; Kim *et al*., 2006).

Leukotriene B₄ (LTB₄) is the most powerful chemotactic and chemokinetic eicosanoid described and promotes adhesion of neutrophils to the vascular endothelium via upregulation of integrins (Hoover *et al.,* 1984). It is also a complete secretagogue for neutrophils, induces their aggregation and increases microvascular permeability. LTB₄ recruits and activates natural killer cells, monocytes and eosinophils. It increases superoxide radical formation (Harrison and Murphy, 1995) and modulates gene expression including production of a number of proinflammatory cytokines and mediators which may augment and prolong tissue inflammation (Ford-Hutchinson, 1990; Showell *et al.,* 1995). LTB₄ also has roles in the induction and management of adaptive immune responses. For example regulation of dendritic cell trafficking to draining lymph nodes (Klaas *et al.,* 2005; Del *Prete et al.,* 2007), Th2 cytokine IL-13 production from lung T cells (Miyahara *et al.,* 2006), recruitment of antigen-specific effector CD8+ T cells (Taube *et al.,* 2006) and activation and proliferation of human B lymphocytes (Yamaoka *et al.,* 1989).

WO2004/106369 describes a soft tick (*Ornithodoros moubata*) derived complement (C) inhibitor OmCI that inhibits both the classical and alternative complement pathways by direct binding to complement component C5 (Nunn *et al.,* 2005). OmCI is derived from the salivary glands of haemotophagous arthropods. It has proven therapeutic potential (Hepburn *et al.,* 2007). It has recently been shown that OmCI binds to eicosanoids, in particular LKs, especially LTB₄.

### Summary of the invention

It has now been shown that OmCI can be modified to reduce or remove leukotriene/hydroxyeicosanoid (LK/E) binding activity. In particular, the present inventors have shown that modifying the OmCI polypeptide at specific residues reduces or removes LTB₄ binding activity. The present invention therefore relates to modified OmCI polypeptides which lack LTB₄ binding activity and to polynucleotides encoding such modified OmCI polypeptides. In particular, the invention relates to OmCI polypeptides which lack LTB₄ binding activity due to the modification of specific residues within the binding pocket of OmCI and to polynucleotides encoding said polypeptides. The invention further relates to OmCI polypeptides that bind to complement components and not LTB₄. Such modified OmCI polypeptides, or polynucleotides encoding such modified OmCI polypeptides act as complement inhibitors and can be used in the prevention and treatment of diseases and conditions mediated by complement, without interfering with the role of LTB₄.

Thus in accordance with one aspect of the present invention, there is provided an OmCI polypeptide which lacks LTB₄ binding activity.

In accordance with a preferred embodiment of the present invention, the OmCI polypeptide comprises:
(a) an amino acid sequence of SEQ ID NO: 3 which has been modified to remove LTB₄ binding activity;
(b) a variant amino acid sequence having at least 60% identity to the amino acid sequence of SEQ ID NO: 3 and which has been modified to remove LTB₄ binding activity;
(c) a variant amino acid sequence of SEQ ID NO: 2 having at least 60% identity to the amino acid sequence between amino acid residues 19 to 168 of SEQ ID NO: 2 and which has been modified to remove LTB₄ binding activity; or
(d) a fragment of the amino acid sequence of (a), (b) or (c) lacking LTB₄ binding activity.

The invention further provides polynucleotides encoding the OmCI polypeptides of the invention.

The invention further provides vectors comprising the polynucleotides of the invention.

The invention further provides host cells comprising the polynucleotides or vectors of the invention.

The invention further provides pharmaceutical compositions comprising an OmCI polypeptide which lacks LTB₄ binding activity; a polynucleotide encoding an OmCI polypeptide which lacks LTB₄ binding activity; or a vector comprising a polynucleotide encoding an OmCI polypeptide which lacks LTB₄ binding activity and a pharmaceutically acceptable carrier.

### Description of the Figures

Fig. 1: Detail from crystal structure of bacterial expressed OmCI (bOmCI) bound to palmitoleic acid (centre of picture).
Fig. 2: Enzyme immunoassay (EIA) showing binding of 12(S)-HETE by 41.2µg bOmCI, pg/mL 12(S)-HETE in solution following 20 min preincubation of 12500µg/mL 12(S)-HETE with or without PBS, OmCI, RaHBP2.
Fig. 3: Absorption spectra of bOMCI and LTB₄. (A) LTB4 in solution (upper line) before addition of OmCI, and re-measurement of same solution (lower line) after addition then removal of the bOmCI:LTB4 complex by ultrafiltation (B) bOmCI:LTB₄ complex (upper line) and bOmCI (lower line) only after concentration to 200µl by ultrafiltation.
Fig. 4: Detail from the crystal structure of bOmCI bound to LTB₄ (centre of picture). Oxygen atoms in LTB₄, at carboxy-group and hydroxyl-groups at C-5 and C-12, are shown. These groups form hydrogen bonds (dotted lines) with amino acids in the binding cavity (see text example 3).
Fig 5: Gas chromatographic analysis of fatty acids present in recombinant OmCI expressed in bacteria. The identity of palmitoleic and elaidic acid was confirmed by mass spectrometry and comparison to reference standards.
Fig 6: Mutant OmCI (yOmCI-F36W and yOmCI-G59W) with the binding pocket blocked by the large amino acid tryptophan shows significantly less binding to LTB₄ than wild type yOmCI at a range of protein concentrations.
Fig 7: Classical pathway haemolytic assay comparing RaHBP2 (negative control tick lipocalin that does not inhibit complement) with wild type recombinant OmCI and mutant yOmCI-F36W and yOmCI-G59W that do not bind LTB₄.
Fig 8: Mutant yOMCI-F36W and yOMCI-G59W with the binding pocket blocked by the large amino acid tryptophan (W) inhibit the classical pathway of complement activation with equal potency to wild type OMCI.
Fig. 9: Binding of radio labelled LTB₄ by OMCI alone and in complex with human C5 is (a) saturable and (b) has similar binding kinetics. Panel (a) is representative of three experiments and shows raw data. Panel (b) is representative of two experiments and shows c.p.m values after subtraction of average negative control c.p.m (n = 16). Logarithmic regression line functions are shown: OMCI, y = 134.67Ln(x) + 521.6, R² = 0.98; OMCI:hC5, y = 132.87Ln(x) + 479.2, R² = 0.97.

### Description of the sequences

SEQ ID NO: 1 is the polynucleotide and encoded protein sequence of OmCI of *O*. *moubata.*

SEQ ID NO: 2 is the amino acid sequence of OmCI *O*. *moubata.*

SEQ ID NO: 3 is the amino acid sequence of amino acids 19 to 168 shown in SEQ ID NO: 2 and is the amino acid sequence of OmCI without the first amino acid sequences of the protein of SEQ ID NO: 2, which is a signal sequence.

SEQ ID NO: 4 and 5 are the polynucleotide and encoded protein sequence and protein sequence respectively of OmCI, modified to change Asn78 to Gln and Asn 102 to Gln, with a codon change from AAT and AAC respectively to CAA, for expression in yeast to avoid hyperglycosylation.

SEQ ID NO: 6 is the polynucleotide and encoded protein sequence of OmCI-F36W mutant of *O*. *moubata*
SEQ ID NO: 7 is the amino acid sequence OmCI-F36W mutant of *O*. *moubata.*

SEQ ID NO: 8 is the amino acid sequence of amino acids 19 to 168 shown in SEQ ID NO: 6 and is the amino acid sequence of OmCI-F36W mutant without the first amino acid sequences of the protein of SEQ ID NO: 6, which is a signal sequence.

SEQ ID NO: 9 is the polynucleotide and encoded protein sequence of OMCI-G59W mutant of *O*. *moubata*
SEQ ID NO: 10 is the amino acid sequence OMCI-G59W mutant of *O*. *moubata.*

SEQ ID NO: 11 is the amino acid sequence of amino acids 19 to 168 shown in SEQ ID NO: 9 and is the amino acid sequence of OMCI-G59W mutant without the first amino acid sequences of the protein of SEQ ID NO: 9, which is a signal sequence.

Thus, the present invention provides an OmCI polypeptide which lacks LTB₄ binding activity or a polynucleotide encoding said OmCI polypeptide. LTB₄ binding activity as used herein refers to the ability of wild-type OmCI to bind to leukotrienes and hydroxyeicosanoids including but not limited to LTB₄, B4 isoleukotrienes and any hydroxylated derivative thereof, HETEs, HPETEs and EETs. The OmCI protein may be a tick-derived complement inhibitor, isolated from the saliva of *O*. *moubata* or may be a functional equivalent thereof, including homologues thereof and fragments of either thereof.

The OmCI protein of the present invention is preferably OmCI from *O.moubata.* This protein was first isolated from the salivary glands of the tick and has been found to inhibit the classical and alternative complement pathways. The amino acid sequence for this protein is shown in SEQ ID NO: 2. A polypeptide according to the invention may include the complete sequence shown in SEQ ID NO: 2 which has been modified to specifically remove LTB₄ binding activity. In the alternative, the polypeptide is provided which does not include the first 18 amino acids of the protein sequence which form a signal sequence which has been modified to specifically remove LTB₄ binding activity. Accordingly, a polypeptide according to the invention can be that of SEQ ID NO: 3, that is amino acids 19 to 168 of the amino acid sequence of SEQ ID NO: 2, additionally modified to remove LTB₄ binding activity.

A variant, such as a homologue, or fragment of the OmCI protein from *O*. *moubata,* which also lacks LTB₄ binding activity, is provided in accordance with the invention. Homologues may include paralogues and orthologues of the OmCI sequence that is set out in SEQ ID NO: 2 or 3, including, for example, the OmCI protein sequence from other tick species including *Rhipicephalus appendiculatus*, *R. sanguineus*, *R. bursa, A. americanum*, *A. caj ennense, A. hebraeum*, *Boophilus microplus, B. annulatus*, *B. decoloratus, Dermacentor reticulatus, D. andersoni, D. marginatus, D. variabilis, Haemaphysalis inermis, Ha. leachii, Ha. punctata, Hyalomma anatolicum anatolicum, Hy. dromedarii*, *Hy. marginatum marginatum*, *Ixodes ricinus*, *I. persulcatus, I. scapularis, I. hexagonus, Argas persicus, A. reflexus, Ornithodoros erraticus, O*. *moubata moubata, O*. *m*. *porcinus,* and *O*. *savignyi,* which have been modified to specifically remove LTB₄ binding activity. The term "homologue" is also meant to include the OmCI protein sequence from mosquito species, including those of the *Culex, Anopheles* and *Aedes* genera, particularly *Culex quinquefasciatus*, *Aedes aegypti* and *Anopheles gambiae;* flea species, such as *Ctenocephalides felis* (the cat flea); horseflies; sandflies; blackflies; tsetse flies; lice; mites; leeches; and flatworms.

In one embodiment, the OmCI polypeptide comprises:
(a) an amino acid sequence of SEQ ID NO: 3 which has been modified to remove LTB₄ binding activity;
(b) a variant amino acid sequence having at least 60% identity to the amino acid sequence of SEQ ID NO: 3 which has been modified to remove LTB₄ binding activity;
(c) a variant amino acid sequence of SEQ ID NO: 2 having at least 60% identity to the amino acid sequence between amino acid residues 19 to 168 of SEQID NO: 2 and which has been modified to remove LTB₄ binding activity; or
(d) a fragment of the amino acid sequence of (a), (b) or (c) lacking LTB₄ binding activity.

Variant polypeptides are those for which the amino acid sequence varies from that in SEQ ID NO: 2 or 3, but which retain the same essential character as the OmCI polypeptides of the invention and which have been modified to remove LTB₄ binding activity.

Typically, polypeptides with more than about 50%, 55%, 60% or 65% identity, preferably at least 60%, at least 70%, at least 80%, at least 90% and particularly preferably at least 95%, at least 97% or at least 99% identity, with the amino acid sequence of SEQ ID NO: 2 or 3, in addition to the one or more LTB₄ binding activity-removing modifications in equivalent positions to those found in the modified OmCI polypeptides of the invention, are considered variants of the protein. Such variants may include allelic variants and the deletion, modification or addition of single amino acids or groups of amino acids within the protein sequence, as long as the peptide maintains the activity of OmCI polypeptides of the invention, and are additionally modified to remove LTB₄ binding activity. The identity of variants of SEQ ID NO: 3 may be measured over a region of at least 50, at least 100, at least 130 or at least 140 or more contiguous amino acids of the sequence shown in SEQ ID NO: 3, or more preferably over the full length of SEQ ID NO: 3.

Amino acid identity may be calculated using any suitable algorithm. For example the UWGCG Package provides the BESTFIT program which can be used to calculate homology (for example used on its default settings) (Devereux et al (1984) Nucleic Acids Research 12, 387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300;Altschul, S, F et al (1990) J Mol Biol 215:403-10.

Software for performing BLAST analyses is publicly available through the National Center for Biotechnology Information (http: //www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The variant sequences typically differ by at least 1, 2, 3, 5, 10, 20, 30, 50 or more mutations (which may be substitutions, deletions or insertions of amino acids). For example, from 1 to 50, 2 to 40, 3 to 30 or 5 to 20 amino acid substitutions, deletions or insertions may be made. The substitutions are preferably conservative substitutions, for example according to Table 1. Amino acids in the same block in the second column and preferably in the same line in the third column may be substituted for each other.

**Table 1: properties of the different amino acid side chains**

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The fragment of the OmCI polypeptide used in the invention is typically at least 50, for example at least 80 or more amino acids in length, up to 90, 100, 120, 130 or 140 amino acids in length, as long as it retains the lack of LTB₄ binding activity of the modified OmCI polypeptide. In a preferred embodiment, the fragment of the OmCI polypeptide retains the complement inhibitor activity shown by OmCI of *O*. *moubata.*

The OmCI polypeptides of the present invention, including variant polypeptides, such as homologues, or fragments thereof lack LTB₄ binding activity. LTB₄ binding activity can be removed by the modification of the amino acid sequence of the OmCI polypeptide. LTB₄ binding activity can also be removed by the modification of the nucleotide sequence of a polynucleotide encoding the OmCI polypeptide of the invention that results in an appropriate modification in the encoded OmCI polypeptide.

The OmCI polypeptide of the present invention can be modified to lack LTB₄ binding activity by the mutation of specific residues within the OmCI amino acid sequence. In one embodiment the mutation of specific amino acid residues is non-conservative, such that one or more amino acid residue of the wild-type OmCI polypeptide is substituted by an amino acid moiety of different polarity. For example, according to Table 1, amino acids in different blocks in the third column may be substituted for each other. In another embodiment, OmCI can be modified by substituting one or more amino acid residue of the wild-type OmCI polypeptide by an amino acid moiety comprising a large side chain to increase steric interference to prevent binding to LTB₄.

In one embodiment, LTB₄ binding activity is removed by the modification of amino acids within the LTB₄ binding pocket of the OmCI polypeptide. Amino acids that are particularly likely to be required for LTB₄ binding include (with reference to SEQ ID NO. 2): Phe36, Arg54, Leu57, Gly59, Val72, Met74, Phe76, Thr85, Trp87, Phe89, Glen 105, Arg107, His119, Asp121, Trp133. In a further embodiment at least 1, 2, 3, 4, 5, 6, 7, 8, 9, 10, 11, 12, 13, 14 or 15 of these amino acids are mutated to remove LTB₄ binding activity. In a preferred embodiment at least one mutation within OmCI is selected from Phe36Trp and Gly59Trp. In another embodiment the modified OmCI polypeptide comprises both the Phe36Trp and Gly59Trp mutations.

In another embodiment, LTB₄ binding activity is removed by the modification of amino acids outside the LTB₄ binding pocket, wherein the modification of said amino acids produces a conformational change in the OmCI polypeptide, inside or outside the LTB₄ binding site, resulting in the loss of LTB₄ binding activity.

The OmCI variant polypeptides, such as homologues, or fragments thereof of the present invention are also modified to lack LTB₄ binding activity. Such variants, in addition to the sequence variations discussed above, are further modified at equivalent positions to those found in the modified OmCI polypeptides of the invention. The amino acids equivalent to the specified positions in OmCI can be determined by aligning the amino acid sequences of the LTB₄ binding pocket of OmCI and the variant and identifying the corresponding amino acid residues.

Methods for determining whether a particular modified OmCI polypeptide has reduced or lacks LK/E binding activity include enzyme immunoassays, light scattering, mass spectrometry, surface plasmon resonance, UV absorption, radioligand or fluorescently labelled ligand binding assays and crystallography. Such methods would be familiar to the person skilled in the art. Examples of specific methods for determining LTB₄ binding of OmCI polypeptides are found in the Examples section.

Typically a modified OmCI polypeptide in accordance with the present invention has reduced LTB₄ binding activity, or lacks this activity. Typically the binding affinity for LTB₄ is reduced by at least 50% compared to unmodified OmCI, for example is reduced by at least 60%,70%, 80%, 85%, 90%, 95%, 98% or 99% or is abolished.

The polypeptides of the invention may also be provided as a fusion protein comprising an OmCI polypeptide genetically or chemically fused to another peptide. The purpose of the other peptide may be to aid detection, expression, separation or purification of the protein. Alternatively the protein may be fused to a peptide such as an Fc peptide to increase the circulating half life of the protein. Examples of other fusion partners include beta-galactosidase, glutathione-S-transferase, or luciferase.

The polypeptides used in the invention may be chemically modified, e.g. post-translationally modified. For example, they may be glycosylated, pegylated, phosphorylated or comprise modified amino acid residues. They may be modified by the addition of histidine residues to assist their purification or by the addition of a signal sequence to promote insertion into the cell membrane. Such modified polypeptides fall within the scope of the term "polypeptide" used herein.

A polypeptide for use in accordance with the invention lacks LTB₄ binding activity. Wild-type OmCI has a propensity to bind to any non-cyclic fatty acid of between 16 and 20 carbon atoms in length. Certain fatty acids, in particular LTB₄ bind more tightly than others. Other fatty acids to which the polypeptide of the invention may bind include arachidonic acid, 12-epi LTB₄, 20-hydroxy LTB₄ and the hydroxyeicosanoids including 12(S)- -hydroxyeicosatetraenoic acid (HETE) and 12(S)- hydroperoxyeicosatetraenoic acid (HPETE). The presence or absence of LTB₄ binding activity or binding activity to other fatty acids of the polypeptide may be determined using techniques such as those discussed above. One such binding assay is exemplified in the Examples. In some embodiments, it may be preferable to select a polypeptide that preferentially lacks binding activity for a specific fatty acid, such as LTB₄. Such preferential lack of binding activity can be determined by suitable assays, for example, competition assays as exemplified in the Examples.

In accordance with some aspects of the invention, preferably, a polypeptide for use in accordance with the invention retains the complement inhibitor activity shown by OmCI of *O*. *moubata.* Preferably, the polypeptide inhibits both the classical and the alternative pathways of complement activation. By inhibit is meant that the effect of the alternative and classical pathways of complement activation is reduced. The ability of a molecule to reduce the effect of the classical complement pathway and the alternative complement pathway can be determined by standard haemolytic assays known in the art such as those described in Giclas et al. (1994) and in WO2004/106369. Preferably, the presence of a complement inhibitor polypeptide of the invention reduces red blood cell lysis in standard haemolytic assays for the classical and alternative pathways of complement activation by at least 20% compared to a standard assay in the absence of a complement inhibitor polypeptide, more preferably by at least 30%, 40%, 50%, 60%, 70% or 80%.

Preferably, the complement inhibitor polypeptide inhibits cleavage of C5 by the C5 convertase in the classical pathway and the C5 convertase in the alternative pathway. The conversion of C5 to C5b by C5 convertase occurs in both the alternative complement pathway and the classical complement pathway. The C5 convertase in the classical pathway is C4b3b2a and the C5 convertase in the alternative pathway is C3b2Bb. The inhibition of C5 cleavage by both these C5 convertases thus inhibits both the classical and alternative pathways of complement activation. The ability of a molecule to inhibit cleavage of C5 by the C5 convertases of the classical and alternative pathways can be determined by standard *in vitro* assays. Preferably, the presence of a complement inhibitor polypeptide reduces cleavage of C5 by the C5 convertases of the classical and alternative pathways by at least 20% compared to a standard assay in the absence of a complement inhibitor polypeptide, more preferably by at least 30%, 40%, 50%, 60%, 70% or 80%. Preferably, the complement inhibitor activity of the polypeptides of the inventions inhibits cleavage of C5 by the C5 convertases of the classical and alternative pathways from a range of mammalian species.

Polypeptides for use in the invention may be in a substantially isolated form. It will be understood that the polypeptide may be mixed with carriers or diluents which will not interfere with the intended purpose of the polypeptide and still be regarded as substantially isolated. A polypeptide for use in the invention may also be in a substantially purified form, in which case it will generally comprise the polypeptide in a preparation in which more than 50%, e.g. more than 80%, 90%, 95% or 99%, by weight of the polypeptide in the preparation is a polypeptide of the invention.

Polypeptides for use in the invention may also be prepared as fragments of such isolated polypeptides. Further, the OmCI polypeptides may also be made synthetically or by recombinant means. For example, a recombinant OmCI polypeptide may be produced by transfecting mammalian, fungal, bacterial or insect cells in culture with an expression vector comprising a nucleotide sequence encoding the polypeptide operably linked to suitable control sequences, culturing the cells, extracting and purifying the OmCI polypeptide produced by the cells.

The amino acid sequence of polypeptides for use in the invention may be modified to include non-naturally occurring amino acids or to increase the stability of the compound. When the polypeptides are produced by synthetic means, such amino acids may be introduced during production. The polypeptides may also be modified following either synthetic or recombinant production.

Polypeptides for use in the invention may also be produced using D-amino acids. In such cases the amino acids will be linked in reverse sequence in the C to N orientation. This is conventional in the art for producing such polypeptides.

A number of side chain modifications are known in the art and may be made to the side chains of the OmCI polypeptides, provided that the polypeptides retain OmCI activity, but lack LTB₄ binding activity.

### Polynucleotides

The present invention provides a polynucleotide encoding an OmCI polypeptide which lacks LTB₄ binding activity. In one embodiment the polynucleotide of the present invention encodes the OmCI protein from *O*. *moubata.* The nucleotide sequence encoding of OmCI from *O.moubata* is shown in SEQ ID NO: 1. A polynucleotide according to the invention may include the complete sequence shown in SEQ ID NO: 1 which has been modified to specifically remove LTB₄ binding activity of the encoded polypeptide.

A polynucleotide encoding a variant, such as a homologue, or fragment of the OmCI protein from *O*. *moubata,* which also lacks LTB₄ binding activity is provided in accordance with the invention. Additional degenerate substitutions may be made and/or substitutions may be made which would result in a conservative amino acid substitution when the modified sequence is translated, for example as shown in Table1 above. Such encoded variants are discussed above and additionally comprise modifications in equivalent positions to those found in the modified OmCI polypeptides of the invention.

A polynucleotide of the invention, including polynucleotides encoding variant polypeptides, such as homologues, or fragments, can typically hybridize to the coding sequence or the complement of the coding sequence of SEQ ID NO: 1 at a level significantly above background. Background hybridization may occur, for example, because of other DNAs present in a DNA library. The signal level generated by the interaction between a polynucleotide of the invention and the coding sequence or complement of the coding sequence of SEQ ID NO: 1 is typically at least 10 fold, preferably at least 100 fold, as intense as interactions between other polynucleotides and the coding sequence of SEQ ID NO: 1. The intensity of interaction may be measured, for example, by radio labelling the probe, e.g. with ³²P. Selective hybridisation may typically be achieved using conditions of medium to high stringency. However, such hybridisation may be carried out under any suitable conditions known in the art (see Sambrook et al, Molecular Cloning: A Laboratory Manual, 1989). For example, if high stringency is required suitable conditions include from 0.1 to 0.2 x SSC at 60°C up to 65°C. If lower stringency is required suitable conditions include 2 x SSC at 60°C.

The coding sequence of SEQ ID NO: 1 may be modified by nucleotide substitutions, for example from 1, 2 or 3 to 10, 25, 50 or 100 substitutions, in addition to encoding one or more LTB₄ binding activity-removing modifications in equivalent positions to those found in the modified OmCI polypeptides of the invention. The polynucleotide of SEQ ID NO: 1 may alternatively or additionally be modified by one or more insertions and/or deletions and/or by an extension at either or both ends. Additional sequences such as signal sequences may also be included or sequences encoding another peptide or protein to aid detection, expression, separation or purification of the protein or encoding a peptide such as an Fc peptide to increase the circulating half life of the protein. Examples of other fusion partners include beta-galactosidase, glutathione-S-transferase, or luciferase.

A nucleotide sequence which is capable of selectively hybridizing to the complement of the DNA coding sequence of SEQ ID NO: 1 will generally have, in addition to encoding one or more LTB₄ binding activity-removing modifications in equivalent positions to those found in the modified OmCI polypeptides of the invention, at least 60%, at least 70%, at least 80%, at least 90%, at least 95%, at least 98% or at least 99% sequence identity to the coding sequence of SEQ ID NO: 3 over a region of at least 20, preferably at least 30, for instance at least 40, at least 60, at least 100, at least 200, at least 420, or most preferably over the full length of SEQ ID NO: 1 or the length of SEQ ID NO: 1 encoding a polypeptide having the sequence shown in SEQ ID NO: 1. Sequence identity may be determined by any suitable method, for example as described above.

Any combination of the above mentioned degrees of sequence identity and minimum sizes may be used to define polynucleotides of the invention, with the more stringent combinations (i.e. higher sequence identity over longer lengths) being preferred. Thus, for example a polynucleotide which has at least 90% sequence identity over 60, preferably over 100 nucleotides forms one aspect of the invention, as does a polynucleotide which has at least 95% sequence identity over 420 nucleotides.

Polynucleotide fragments will preferably be at least 20, for example at least 25, at least 30 or at least 50 nucleotides in length. They will typically be up to 100, 150, 250 or 400 nucleotides in length. Fragments can be longer than 400 nucleotides in length, for example up to a few nucleotides, such as five, ten or fifteen nucleotides, short of the coding sequence of SEQ ID NO: 1.

The OmCI polynucleotides of the present invention, including polynucleotides encoding variant polypeptides, such as homologues, or fragments, all encode polypeptides that have reduced or lack LTB₄ binding activity. Such polynucleotide variants, in addition to the sequence variations discussed above, are further modified at equivalent positions to those found in the modified OmCI polypolynucleotides of the invention, so that they encode polypeptides lacking LTB₄ binding activity. The nucleic acids equivalent to the specified positions in OmCI can be determined by aligning the nucleic acid sequences of the modified OmCI and the variant and identifying the corresponding nucleic acid residues.

LTB4 binding activity can be removed by the modification of the nucleotide sequence of a polynucleotide encoding the OmCI polypeptide of the invention that results in an appropriate modification in the encoded OmCI polypeptide.

The OmCI polynucleotide of the present invention can be modified to mutate specific residues within the amino acid sequence of the encoded OmCI polypeptide, resulting in removal of LTB4 binding activity. In one embodiment non-degenerate substitutions may be made in the polynucleotide of the invention, which would result in a non-conservative amino acid substitution when the modified sequence is translated, for example as shown in Table 1 above.

In one embodiment, LTB4 binding activity is removed by the modification of the polynucleotide of the invention to mutate amino acids within the LTB4 binding pocket of the encoded OmCI polypeptide. Amino acids that are particularly likely to be required for LTB₄ binding include (with reference to SEQ ID NO. 2): Phe36, Arg 54, Leu57, Gly59, Val72, Met74, Phe76, Thr85, Trp87, Phe89, Gln105, Arg107, His119, Asp121, Trp133. In a further embodiment the polynucleotide of the invention is modified so that the encoded OmCI polypeptide is mutated at least 1, 2, 3, 4, 5, 6, 7, 8, 9, 10, 11, 12, 13, 14 or 15 of these amino acids to remove LTB4 binding activity. In a preferred embodiment the polynucleotide of the invention is modified so that at least one mutation within the encoded OmCI polypeptide is selected from Phe36Trp and Gly59Trp.

A polynucleotide of the invention may be used to treat or prevent a disease or condition mediated by complement. Typically the polynucleotide is DNA. However, the polynucleotide may be a RNA polynucleotide. The polynucleotide may be single or double stranded, and may include within it synthetic or modified nucleotides.

Polynucleotides for use in the invention may be produced recombinantly, synthetically, or by any means available to those of skill in the art. They may also be cloned by standard techniques. The polynucleotides are typically provided in isolated and/or purified form.

In general, short polynucleotides will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using PCR (polymerase chain reaction) cloning techniques. This will involve making a pair of primers (e.g. of about 15-30 nucleotides) to a region of the OmCI gene which it is desired to clone, bringing the primers into contact with DNA obtained from an arthropod cell performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Such techniques may be used to obtain all or part of the OmCI gene sequence described herein. Although in general the techniques mentioned herein are well known in the art, reference may be made in particular to Sambrook *et al.* (1989).

OmCI polynucleotides as described herein have utility in production of the polypeptides for use in the present invention, which may take *place in vitro, in vivo* or *ex vivo.* The polynucleotides may be used as therapeutic agents in their own right or may be involved in recombinant protein synthesis.

The polynucleotides for use in the invention are typically incorporated into a recombinant replicable vector. The vector may be used to replicate the nucleic acid in a compatible host cell. Therefore, polynucleotides for use in the invention may be made by introducing an OmCI polynucleotide into a replicable vector, introducing the vector into a compatible host cell and growing the host cell under conditions which bring about replication of the vector. The host cell may, for example, be an E. *coli* cell.

Preferably the vector is an expression vector comprising a nucleic acid sequence that encodes an OmCI polypeptide. Such expression vectors are routinely constructed in the art of molecular biology and may for example involve the use of plasmid DNA and appropriate initiators, promoters, enhancers and other elements, such as for example polyadenylation signals, which may be necessary and which are positioned in the correct orientation in order to allow for protein expression. The coding sequences may also be selected to provide a preferred codon usage suitable for the host organism to be used. Other suitable vectors would be apparent to persons skilled in the art. By way of further example in this regard we refer to Sambrook *et al.* (1989).

Preferably, a polynucleotide for use in the invention in a vector is operably linked to a control sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence, such as a promoter, "operably linked" to a coding sequence is positioned in such a way that expression of the coding sequence is achieved under conditions compatible with the regulatory sequence.

The vectors may be for example, plasmid, virus or phage vectors provided with an origin of replication, optionally a promoter for the expression of the said polynucleotide and optionally a regulator of the promoter. The vector is typically adapted to be used *in vivo.*

Promoters and other expression regulation signals may be selected to be compatible with the host cell for which expression is designed. Mammalian promoters, such as β-actin promoters, may be used. Tissue-specific promoters are especially preferred. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, adenovirus, HSV promoters (such as the HSV IE promoters), or HPV promoters, particularly the HPV upstream regulatory region (URR). Viral promoters are readily available in the art.

The vector may further include sequences flanking the polynucleotide giving rise to polynucleotides which comprise sequences homologous to eukaryotic genomic sequences, preferably mammalian genomic sequences. This will allow the introduction of the polynucleotides of the invention into the genome of eukaryotic cells by homologous recombination. In particular, a plasmid vector comprising the expression cassette flanked by viral sequences can be used to prepare a viral vector suitable for delivering the polynucleotides of the invention to a mammalian cell. Other examples of suitable viral vectors include herpes simplex viral vectors and retroviruses, including lentiviruses, adenoviruses, adeno-associated viruses and HPV viruses. Gene transfer techniques using these viruses are known to those skilled in the art. Retrovirus vectors for example may be used to stably integrate the polynucleotide giving rise to the polynucleotide into the host genome. Replication-defective adenovirus vectors by contrast remain episomal and therefore allow transient expression.

### Diseases and Conditions

Wild-type OmCI is known to bind to both complement and LK/E molecules such as LTB₄. The present inventors have found that OmCI can be modified to specifically remove LTB₄ binding activity. The present inventors have identified specific residues within the binding pocket of OmCI that can be mutated to remove LTB₄ binding activity but have no effect on the ability of OmCI to bind to complement components. LTB4 is the most powerful chemotatic and chemokinetic eicosanoid described and promotes adhesion of neutrophils to the vascular endothelium via up-regulation of integrins. LTB₄ induces aggregation of neutrophils and through a variety of processes plays a role inflammation.

Thus, the specific removal of LTB₄ binding activity provides the opportunity to use such modified OmCI polypeptides and polynucleotides encoding such modified OmCI polypeptides for treating diseases and conditions mediated by complement, without interfering with the role of leukotrienes and eicosanoids in the immune system.

The treatment of pathological condition(s) wherein complement activation is inhibited but LTB4 or other fatty acids that would be bound by wild type OmCI a represent is desirable when:
a) a disease or condition is mediated by complement and LTB4 has no role; or
b) a disease or condition is mediated by complement and where LTB4, or the neutrophils it recruits, have a beneficial role rather than exacerbating the disease or condition. An example of such a specific disorder that can be treated in accordance with the present invention is the treatment of cancer where C5a receptor blockade has recently been shown to impair tumor growth in an animal model but where neutrophils present anti-tumour activity via their recruitment to sites of inflammation.
c) a patient, such as an immunosuppressed individual, would benefit from inhibiting one immune defence mechanisms (i.e. complement) rather than two (complement and LTB4). An example of such a specific disorder that can be treated in accordance with the present invention is the treatment of cancer wherein the patient undergoes chemotherapy and so has a suppressed neutrophil migatory response, making them susceptible to bacterial infections. Here the presence of LTB₄ reduces the risk of secondary infections.

Examples of specific disorders that can be treated in accordance with the present invention include age-related macular degeneration (AMD), Alzheimer's disease, allergic encephalomyelitis, allotransplatation, arthritis of various sorts including rheumatoid arthritis, asthma, adult respiratory distress syndrome, burn injuries, cancer, Crohn's disease, dermatomyositis, glomerulonephritis, haemolytic anaemia, haemodialysis, hereditary angioedema, idiopathic membranous nephropathy, in utero growth restriction (IUGR), ischaemia reperfusion injuries, motor neuron disease, multiple system organ failure, multiple sclerosis, myasthenia gravis, myocardial infarction, nephritis, pemphigoid, post cardiopulmonary bypass, psoriasis, septic shock, spontaneous miscarriage, stroke, systemic lupus erythematosus, uveitis, vascular leak syndrome and xenotransplantation.

In a preferred embodiment, specific disorders that can be treated in accordance with the present invention include age-related macular degeneration (AMD), Alzheimer's disease, allergic encephalomyelitis, allotransplatation, adult respiratory distress syndrome, burn injuries, cancer, dermatomyositis, glomerulonephritis, haemolytic anaemia, haemodialysis, hereditary angioedema, idiopathic membranous nephropathy, in utero growth restriction (IUGR), ischaemia reperfusion injuries, motor neuron disease, multiple system organ failure, myasthenia gravis, pemphigoid, post cardiopulmonary bypass, septic shock, spontaneous miscarriage, vascular leak syndrome and xenotransplantation.

### Therapy and Prophylaxis

Disclosed herein is the use of OmCI polypeptides and polynucleotides to treat or prevent a disease or condition mediated by complement. Treatment may be therapeutic or prophylactic.

The OmCI polypeptide or polynucleotide may be administered to an individual in order to prevent the onset of one or more symptoms of the disease or condition. In this embodiment, the subject may be asymptomatic. The subject may have a genetic predisposition to the disease. A prophylactically effective amount of the polypeptide or polynucleotide is administered to such an individual. A prophylactically effective amount is an amount which prevents the onset of one or more symptoms of a disease or condition.

A therapeutically effective amount of the OmCI polypeptide or polynucleotide is an amount effective to ameliorate one or more symptoms of a disease or condition. Preferably, the individual to be treated is human.

The OmCI polypeptide or polynucleotide may be administered to the subject by any suitable means. The polypeptide or polynucleotide may be administered by enteral or parenteral routes such as via oral, buccal, anal, pulmonary, intravenous, intra-arterial, intramuscular, intraperitoneal, intraarticular, topical or other appropriate administration routes.

The OmCI polypeptide or polynucleotide may be administered to the subject in such a way as to target therapy to a particular site.

The formulation of any of the polypeptides and polynucleotides mentioned herein will depend upon factors such as the nature of the polypeptide or polynucleotide and the condition to be treated. The polypeptide or polynucleotide may be administered in a variety of dosage forms. It may be administered orally (e.g. as tablets, troches, lozenges, aqueous or oily suspensions, dispersible powders or granules), parenterally, subcutaneously, intravenously, intramuscularly, intrastemally, transdermally, topically or by infusion techniques. The polypeptide or polynucleotide may also be administered as suppositories. A physician will be able to determine the required route of administration for each particular patient.

Typically the polypeptide or polynucleotide is formulated for use with a pharmaceutically acceptable carrier or diluent and this may be carried out using routine methods in the pharmaceutical art. The pharmaceutical carrier or diluent may be, for example, an isotonic solution. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for intravenous or infusions may contain as carrier, for example, sterile water or preferably they may be in the form of sterile, aqueous, isotonic saline solutions.

For suppositories, traditional binders and carriers may include, for example, polyalkylene glycols or triglycerides; such suppositories may be formed from mixtures containing the active ingredient in the range of 0.5% to 10%, preferably 1% to 2%.

Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the pharmaceutical composition is lyophilised, the lyophilised material may be reconstituted prior to administration, e.g. a suspension. Reconstitution is preferably effected in buffer.

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

Pharmaceutical compositions suitable for delivery by needleless injection, for example, transdermally, may also be used. The compositions according to the invention may be presented in all dosage forms normally used for topical application, in particular in the form of aqueous, aqueous-alcoholic or, oily solutions, of dispersions of the lotion or serum type, of anhydrous or lipophilic gels, of emulsions of liquid or semi-solid consistency of the milk type, obtained by dispersing a fatty phase in an aqueous phase (O/VV) or vice versa (VV/O), or of suspensions or emulsions of soft, semi-solid consistency of the cream or gel type, or alternatively of microemulsions, of microcapsules, of microparticles or of vesicular dispersions to the ionic and/or nonionic type. These compositions are prepared according to standard methods.

They may also be used for the scalp in the form of aqueous, alcoholic or aqueous-alcoholic solutions, or in the form of creams, gels, emulsions or foams or alternatively in the form of aerosol compositions also containing a propellant agent under pressure.

The amounts of the different constituents of the compositions according to the invention are those traditionally used in the fields in question.

A therapeutically effective amount of polypeptide or polynucleotide is administered. The dose may be determined according to various parameters, especially according to the polypeptide or polynucleotide used; the age, weight and condition of the patient to be treated; the route of administration; and the required regimen. Again, a physician will be able to determine the required route of administration and dosage for any particular patient. A typical daily dose is from about 0.001 to 50mg per kg, preferably from about 0.01mg/kg to 10mg/kg of body weight, according to the activity of the polypeptide, the age, weight and conditions of the subject to be treated, the type and severity of the disease and the frequency and route of administration. Preferably, daily dosage levels are from 0.5mg to 2g. Lower dosages may be used for topical administration.

The modified OmCI nucleotide sequences described above and expression vectors containing such sequences can also be used as pharmaceutical formulations as outlined above. Preferably, the nucleic acid, such as RNA or DNA, in particular DNA, is provided in the form of an expression vector, which may be expressed in the cells of the individual to be treated. The formulations may comprise naked nucleotide sequences or be in combination with cationic lipids, polymers or targeting systems. The formulations may be delivered by any available technique. For example, the nucleic acid may be introduced by needle injection, preferably intradermally, subcutaneously or intramuscularly. Alternatively, the nucleic acid may be delivered directly across the skin using a nucleic acid delivery device such as particle-mediated gene delivery. The nucleic acid may be administered topically to the skin, or to mucosal surfaces for example by intranasal, oral, intravaginal or intrarectal administration.

Uptake of nucleic acid constructs may be enhanced by several known transfection techniques, for example those including the use of transfection agents. Examples of these agents include cationic agents, for example, calcium phosphate and DEAE-Dextran and lipofectants, for example, lipofectam and transfectam. The dosage of the nucleic acid to be administered can be altered. Typically the nucleic acid is administered in the range of 1pg to 1mg, preferably to 1pg to 1µg nucleic acid for particle mediated gene delivery and 10µg to 1mg for other routes.

### Examples

### Example 1: Wild-type OmCI binds 12(S)-HETE (12(S)-hydroxyeicosatetraenoic acid) in a competitive ELISA

### Background:

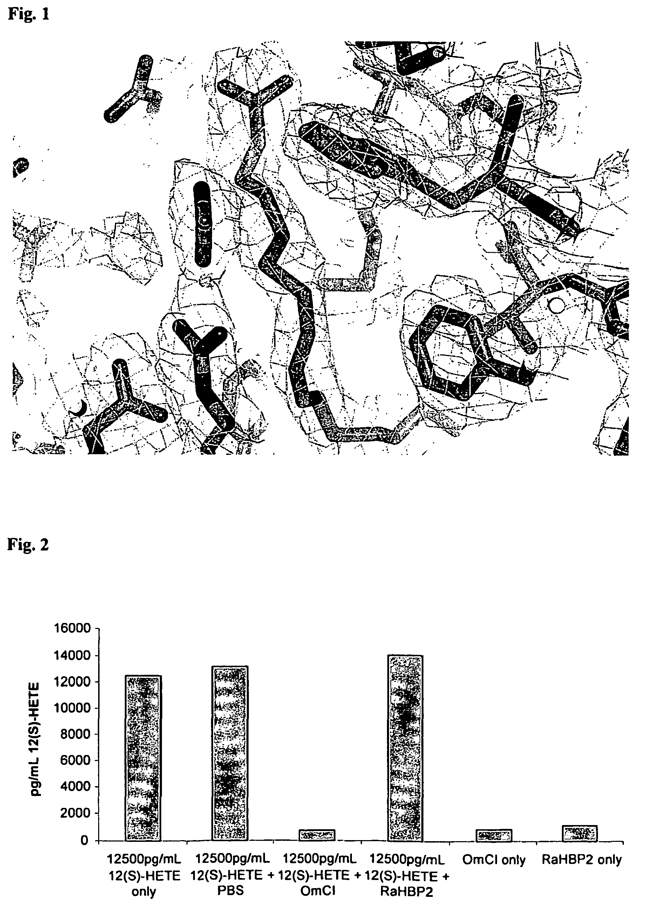

OmCI binds to fatty acids (Figure 1). Mass spectroscopy shows that ricinoleic acid (C₁ₛH₃₄O₃) and palmitoleic acid (C₁₆H₃₀O₂) are the predominant forms found **in** OmCI expressed in *P. methanolica* and *E. coli* respectively. However, the true physiological ligands are more likely to be one or more of the many host cell membrane derived eicosanoids which mediate inflammation, oxidative stress and cell signalling.

Competitive enzyme immunoassays (EIAs) from Assay Designs Inc. are available for the quantification of a number of the eicosanoids. One such EIA kit uses a polyclonal antibody to 12(S)-HETE to bind 12(S)-HETE labelled with alkaline phosphatase and competing unlabelled 12(S)-HETE in the sample or standards of known concentration. After simultaneous incubation at room temperature and capture of the antibody on the plate, the excess reagents are washed away, the substrate added and reaction measured by microplate reader. The higher the concentration of 12(S)-HETE in sample or standard the lower the absorbance reading, because the unlabelled fatty acid competes for binding with the alkaline phosphatase labelled molecule.

We hypothesised that OmCI would compete for binding with the eicosanoid specific antibodies used in the immunoassays. 12(S)-hydroxyeicosatetraenoic acid (12(S)-HETE) was chosen to test this idea since it is (perhaps) the eicosanoid with the most similar physicochemical characteristics to ricinoleic acid (which, from our crystallographic data, is predicted to bind more tightly than palmitoleic acid). Among other effects, 12(S)-HETE has been shown to be chemotactic and chemokinetic for polymorphonuclear leukocytes and vascular smooth muscle cells.

### Methods:

12(S)-HETE EIA Kit was from Assay Designs (Cat. No. 900-050). OmCI stocks used were expressed either in yeast (yOmCI) or bacteria (bOmCI). Both stocks were ≥98% pure, 8.3mg/mL in phosphate buffered saline pH 7.2 (PBS). The negative control tick histamine binding protein RaHBP2, which is also a lipocalin (Paesen *et al.,* 1999), was expressed in bacteria and was also ≥98% pure, 8.3mg/mL in PBS. 12(S)-HETE standard was diluted to 50000, 12500, 3125, 781, 195pg/mL in the assay buffer supplied with the kit. 100µl of the 12500, 3125 and 0pg/mL solutions were mixed with ≤9µl of phosphate buffered saline pH 7.2 (PBS) or solutions of OmCI or RaHBP2 in PBS. The mixtures were incubated at room temperature for 20 minutes then used in the 12(S)-HETE immunoassay in accordance with the manufacturers instructions. The absorbance readings of the treated samples were compared with a standard curve to estimate the concentration of 12(S)-HETE available in solution for binding by the anti-12(S)-HETE polyclonal antibody.

### Results:

bOmCI but not RaHBP2 decreases the amount of 12(S)-HETE available in solution for antibody binding suggesting bOmCI binds directly to 12(S)-HETE (Fig. 2). PBS and both the bOmCI and RaHBP2 purified protein preps appear to contain some (≤ 1000pg/mL) 12(S)-HETE. Similar methods were used to show that OmCI binds to LTB₄ (data not shown).

### Discussion:

These initial results with the bacterially expressed protein suggest OmCI can bind fatty acids that are longer (C20) and have a greater number of unsaturated bonds (four) than either palmitoleic (C16 and 1 double bond) or ricinoleic acid (C18 and 1 double bond). Furthermore 12(S)-HETE does not have a double bond at C9 - C10 which was predicted to be important for ligand binding. The results also suggest that palmitoleic acid can be displaced from the binding pocket of bOmCI by 12(S)-HETE. Although caution is needed with this assumption, since OmCI was used in large molar excess (∼1000 - 4000-fold) and it is possible that a proportion of the purified bOmCI is not occupied by any ligand.

### Example 2: LTB₄ binding by wild-type OmCI is evident by absorbance

### Background:

Leukotrienes have characteristic, strong, UV absorption spectra due to their conjugated double bond systems (the triene chromophore). In aqueous media LTB₄ has a peak absorbance at 271nm and 'shoulders' at 262nm and 282.5nm. Protein peak absorbance is at 280nm. OmCI bound to LTB₄ should exhibit increased UV absorbance at around 280nm, compared to the protein on its own, and LTB₄'s characteristic shoulders 10nm either side of the peak absorbance.

### Method:

bOmCI (4.5mg) was incubated with 1.8mL LTB₄ (50ng/µL stock in pure ethanol, Biomol International) in 39mL PBS at room temperature with shaking for 10 minutes. This mixture is a 1:1 molar ratio between OmCI and LTB₄. The mixture was concentrated to 200µl in Vivaspin (Sartorious) 5kDa cut off ultrafiltration device. The retentate was washed with a further 30mL of PBS and concentrated to 200µl. In parallel, the same amount (4.5mg) of bOmCI was incubated with 1.8ml ultrapure ethanol in 39mL PBS, then concentrated and washed as described above. The final volume of the concentrated proteins was 200µl. UV absorption spectra of the proteins were examined using a Nanodrop ND-1000 Spectrophotometer.

### Results:

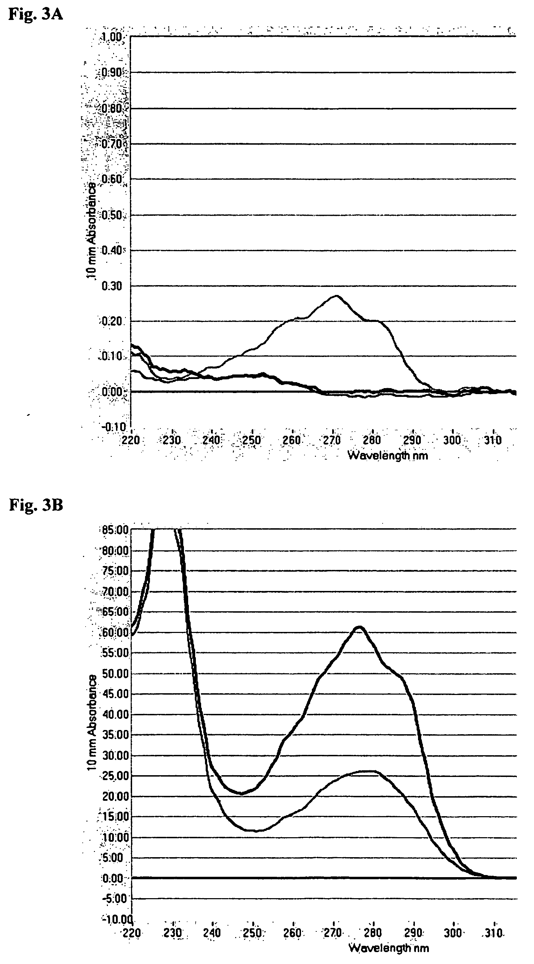

The spectra obtained are shown in Fig. 3. LTB₄ alone has the characteristic absorbance peaks expected in phosphate buffered saline pH 7.4 with peaks at 271, 261 and 281nm (Fig. 3A). The absorption spectra of bOmCI incubated with LTB₄, washed extensively to remove residual LTB₄, has shoulders indicative of LTB₄ binding and peak absorbance is significantly higher than bOmCI incubated with pure ethanol (Fig. 3B). This indicates that bOmCI selectively binds LTB₄ and removes it from solution. Indeed, no LTB₄ is detectable in the flow through from the initial ultrafiltation step (Fig 3A) which indicates that (within the limits of detection) all the LTB₄ added to initial mixture was bound by the bOmCI.

Significant changes in the UV spectra of LTB₄ bound by bOmCI were observed. The UV maximum exhibited a +6nm bathochromic (red shift) to 277, 267 and 287nm (Fig. 3A and B). The shift is most likely caused by dispersion interactions between the conjugated leukotriene and bOmCI amino acids. This is consistent with the triene chromophore being completely encompassed by the protein. Similar interactions will cause hypochromism of UV absorption by the triene chromophore. This was not measured directly, but it is notable that peak absorption expected from input LTB₄ concentrated to 200µl (final volume of concentrated protein) would be 55.8 (calculation 41.32ml/0.2ml x 0.27 10mm Absorbance) whereas total peak absorption of LTB₄ bound to bOmCI was approximately 35.07 (calculation peak 10mm absorption of bOmCI:LTB4 minus peak absorption bOmCI i.e. 61.19 - 26.12). Assuming minimal losses of protein, the calculation implies hypochromism.

### Example 3: Crystallographic structural data shows LTB₄ in the binding pocket of wild-type bOmCI.

### Method:

bOmCI protein loaded with LTB₄ was made as described above (Example 2), then concentrated to 25mg/mL, buffer exchanged to Tris-HCl pH 7, 30mM NaCL and used to grow crystals. A diffraction dataset was collected from a P2₁ OmCI:LTB4 monoclinic crystal (a=41.76 Å b=112.81 Å c=62.40 Å β=101.89°, 4 copies/asymmetric unit) in July 2008 on BM14@ESRF. The data have been processed to 2.0 Å resolution, the structure was initially determined by molecular replacement and the OmCI:LTB4 model built and refined to R=20.7 R_{free}=23.7, rmsd_{bonds}=0.005, rmsd_{angles}=0.9.

### Results:

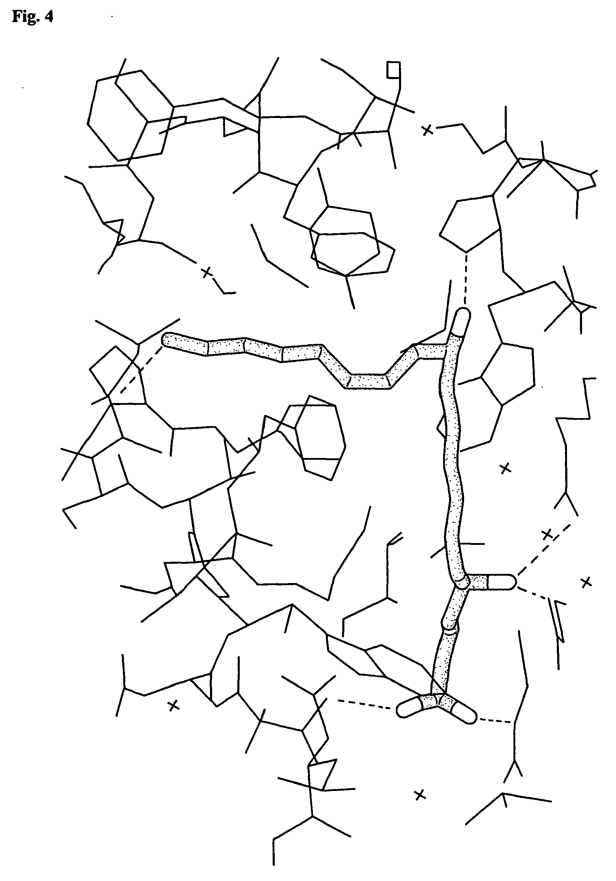

Figure 4 shows a ball and stick representation of LTB₄ in the bOMCI binding pocket. The following residues are directly involved in binding to LTB₄:
- Arg54,Thr85,Trp87: these residues hydrogen bond the head (carboxy group) of LTB₄; modifications of these residues can be engineered to bind ligands that differ in the chemistry of the head group
- The hydrophobic body of the LTB₄ contacts the hydrophobic side chains of the pocket: Phe36, Tyr43, Pro61, Leu70, Val72, Phe76, Leu57, Met74, Arg107, Phe89, Trp133, Trp87, Gly59
- Arg107 and Gln105 recognise the -OH at LTB₄ carbon 5 (C5)
- His119 and Asp121 recognise the -OH at LTB₄ carbon 12 (C12)
Ricinoleic acid lacks the -OH group at carbon 5, has only a single double bond between C9 and C10 and is two carbon atoms shorter than LTB₄. The major structural differences between OMCI bound to ricinoleic acid bound compared to LTB₄ are in the region of the 132-142 loop that is necessary for C5 inhibition (Mans and Ribeiro, 2008). The differences can be summarised as follows:
- Glu141 and His164 side chains flip (these changes at His164 and Glu141 are related via two hydrogen bonds from the side chains of Arg47 and Arg148); as a result of these side chain flips, the His164:Asp136 salt bridge is lost and the 132-142 loop is pulled in via a side chain flip of His 117, which hydrogen bonds to G139, and loss of the bridging water. This conformational change induced by LTB₄ binding may have an effect of the binding kinetics of OmCI to C5 but we do not presently have any direct evidence for this.
- The second region which shows a minor rearrangement is 155-159 No direct contact exists between the C5-inhibitory region 132-142 and the pocket. The 132-142 loop structure is the same in all four copies in the asymmetric unit despite this loop being in three different crystal packing environments across the 4 copies: so it is possible that the differences with relation to the ricinoleic acid structure be due to subtle propagation of structure from ligand to loop via an intermediate layer of small changes.

### Example 4: Fatty acids present in recombinant OmCI expressed in bacteria

### Background:

It is known that ricinoleic acid (47%), palmitic acid methyl ester (21 %), and stearic acid methyl ester (11%) are the predominant fatty acids bound by recombinant OmCI expressed in yeast. It has also recently been shown that OmCI can bind to 12(S)-hydroxyeicosateranoic acid (HETE) and most strongly to LTB4. Homologues of OmCI are known to bind to arachidonic acid. The present inventors have shown that the OmCI expressed in bacteria binds yet other fatty acids.

### Method:

The crude OMCI protein extract (CHCl₃, 130 µl) was evaporated to dryness at room temperature with a gentle stream of nitrogen. A few drops of an ethereal solution of diazomethane were added to convert the free acid into the methyl ester. After 10 min the solvent and excess of diazomethane were removed by a stream of nitrogen and the sample was taken up in dichloromethane (50 µl). After further concentration to 10 µl the sample was directly used for GC-MS analysis. The reference fatty acid was likewise converted to the methyl ester by diazomethane prior to GC-MS analysis on a TraceMS (ThermoFinnigan, D-63329 Egelsbach, Germany) equipped with fused silica Alltech EC5 (D-82008 Unterhaching, Germany) capillary (15 m x 0.25 mm, 0.25 µm) using He at 1.5 ml min⁻¹ as the carrier gas. Samples (1 µl) were injected in the split less mode (1 min) and separated under programmed conditions starting at 60 °C (1 min) followed by heating with 10 °C min⁻¹ to 180 °C, and with 4 °C min⁻¹ to 280 °C maintained for 2 min. Full scan spectra were measured in electron impact (EI) mode at 70 eV, with a source temperature of 200 °C, transfer line at 280 °C, and an emission current of 250 µA. The instrument was operated between m/z 50 and m/z 540 at 2 scans sec⁻¹. The presence of palmitoleic acid and elaidic acid methyl esters was confirmed by comparison with authentic samples.

### Results:

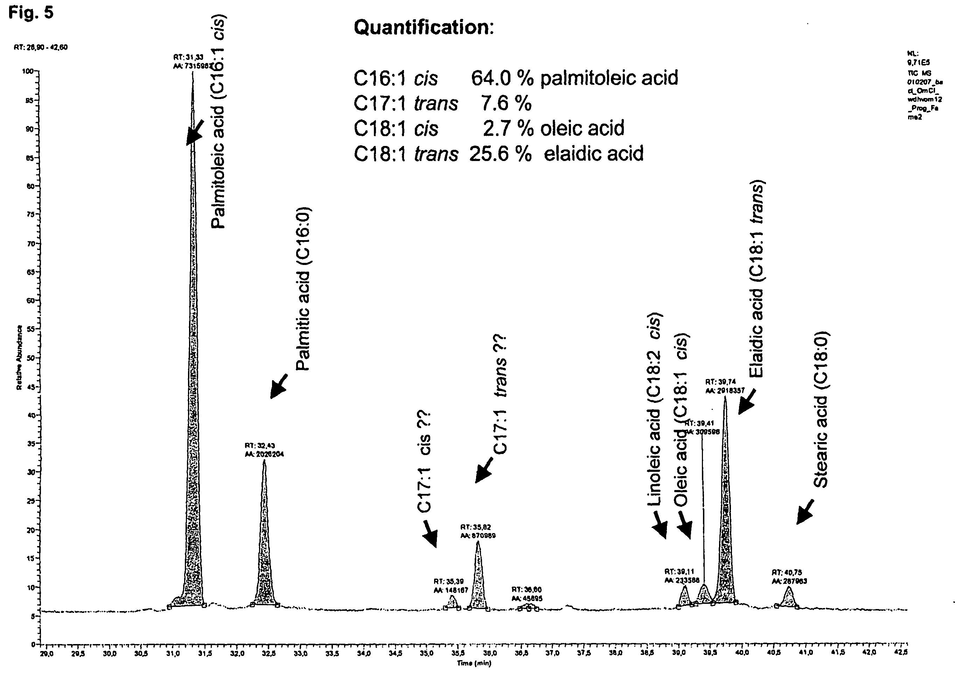

For identification of the protein-associated fatty acid the esterified sample was analyzed by gas chromatography (Fig. 5) and mass spectroscopy (not shown). The major component (64%) appears to be the cis-isomer of palmitoleic acid (C16:1 *cis*). There is a minor component (7.6%) of a C17:1 monounsaturated fatty acid with both *cis-* and *trans-*isomers present. The final compounds are C18:1 with the transisomer prevailing. Oleic acid (C18:1 cis) is a minor compound (2.7%). The major C18:1 component is probably elaidic however the retention time was not perfectly identical to the reference whereas the oleic acid was. What we termed elaidic acid may actually be vaccenic acid (C18:1 trans but with the double bond at C11 instead of C9 in elaidic- or oleic acid). The occurrence of *trans*-fatty acids is consistent of the bacterial origin of OMCI. The saturated acids are not from the sample but were already present in the silylation reagent (C16:0 and C18:0) and were not included in the quantification.

### Discussion:

OmCI can bind to a variety of fatty acids between 16 and 20 carbons in length that vary in number and position of their unsaturated bonds and hydroxyl groups. A variety of fatty acids are present in recombinantly expressed OmCI. The identity of the fatty acids present in the binding cavity depends upon their concentration in the protein solution and the binding specificity for each fatty acid.

### Example 5: Site directed mutagenesis of specific residues within the binding pocket of OmCI ablates LTB₄ binding.

### Background

LTB₄ is enclosed by OmCI within a binding pocket. The pocket can be blocked, and LTB₄ binding prevented, by using site directed mutagenesis to insert a large residue, such as tryptophan, in place of a smaller one present in the wild type protein. The binding of LTB₄ to OmCI can be measured by a variety of techniques including competitive enzyme immunoassays (EIAs) available for the quantification of eicosanoids. In the assay OmCI competes for binding with the LTB₄ specific antibodies used in the EIA.

### Method:

PCR site directed mutagenesis was used to change phenyalanine 36 for tryptophan (yOmCI-F36W) and, separately, glycine 59 for tryptophan (yOmCI-G59W). The mutant proteins were expressed in yeast (*Pichia methanolica*), purified to homogeneity (>95% pure) and concentrations determined by absorption. Binding of the mutants to LTB₄ was compared to wild type using Assay Design Inc EIA kits (see example 1).

### Results:

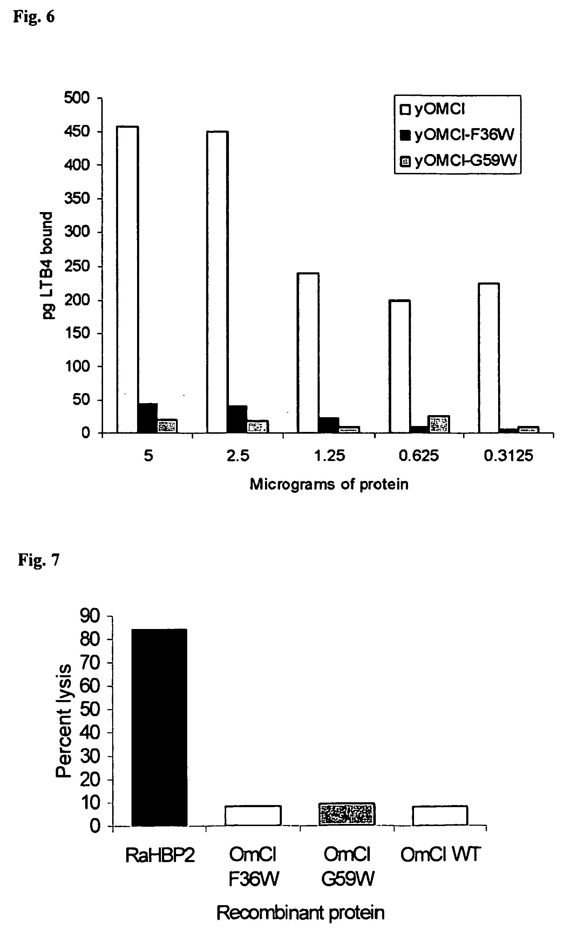

As shown in Fig. 6, yOmCI-F36W and yOmCI-G59W showed significantly less binding to LTB₄ than wild type yOmCI. Modelling indicates that the mutations block the binding pocket. It is likely that these mutants prevent binding of all fatty acids and not just LTB4.

### Discussion:

The ability of OmCI to bind to LK/E molecules such as LTB₄ can be reduced or removed by mutating key residues within the binding pocket of OmCI. This binding site is distinct to the complement binding site of OmCI. Therefore, by specifically removing LK/E binding activity, modified OmCI polypeptides can be used to target complement-mediated diseases and conditions without interfering with the action of LK/E.

### Example 6: Site directed mutants of OmCI, which are unable to bind LTB₄, can inhibit complement

### Background

Mutation of the residues that prevent LTB₄ binding might prevent yOmCI-F36W and yOmCI-G59W acting as complement inhibitors. We therefore compared inhibition of the classical pathway of complement by wild type OmCI and the yOmCI-F36W and yOmCI-G59W mutants.

### Method:

Sheep blood cells were from Tissue Culture Services. Haemolysin was obtained from Sigma. Guinea pig sera were from in house animals. Five ml of fresh sheep blood in Alsever's solution (1:1 vol/vol) were washed once in 50ml Gelatin veronal barbital-EDTA (GVB-EDTA) and three times in 50ml GVB²⁺ buffer (GVB buffer with Mg²⁺ and Ca²⁺). The blood was diluted to a concentration of 1 x 10⁹ cells ml⁻¹. The erythrocytes were sensitised using rabbit haemolysin, titrated as described (Coligan, 1994). Assays were carried in a total volume of 100µl using 100 µl 1:320 of diluted guinea pig sera in GVB²⁺ as a source of complement and 50µl 2x10⁸ sensitised erythrocytes (EA) in accordance with standard protocols (Giclas, 1994). Five micrograms of the recombinant proteins OmCI or PBS (5µl) was added last, and the reactions incubated with shaking (500rpm) at 37°C. After 30 min whole cells were spun down 12000 x g for 5 seconds and hemolysis measured spectrophotometrically at 412nm (Coligan, 1994). All assays were carried out in triplicate.

### Results:

As shown in Fig.7, yOmCI-F36W and yOmCI-G59W inhibited the classical pathway of complement activation as potently as wild type OmCI at the concentration (5 micrograms per reaction) used.

In a further experiment, sheep red blood cells were sensitised using rabbit haemolysin (Sigma), washed in GVB²⁺ buffer (GVB buffer with Mg²⁺ and Ca²⁺) and adjusted to 1 x 10⁹ cells ml⁻¹. Assays were carried in a total volume of 100µl using 50µl 1:320 diluted guinea pig serum in GVB²⁺ as a source of complement and 50µl 2x10⁸ activated erythrocyte (EA) cells ml⁻¹. Five microliters of recombinant wild type or mutant OMCI or RaHBP2 control protein diluted in PBS was added last, and reactions incubated at 37°C for 30min. The whole cells were then spun down 12000 x g for 5 seconds and hemolysis measured spectrophotometrically at 412nm. Percent lysis of samples was calculated using the absorbance value for 100% cell lysis caused by adding water in place of GVB²⁺ buffer to the EA.

### Results:

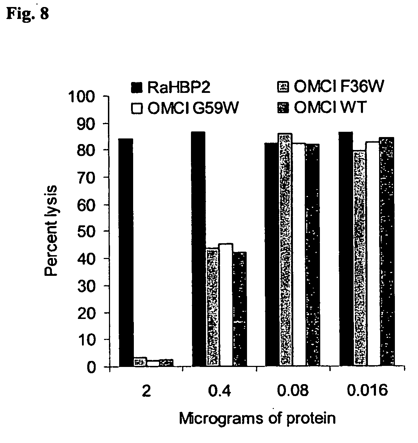

Fig. 8 shows that there is no difference in the inhibition of the classical pathway of complement activation by wild type OMCI and OMCI that is unable to bind LTB₄. This suggests LTB₄ binding has no effect on OMCI binding to C5.

### Discussion:

The ability of the two mutant forms of OmCI (yOmCI-F36W and yOmCI-G59W) to inhibit complement implies that fatty acid binding is not necessary for OmCI to inhibit complement. This is supported by the crystallographic structural data which show only subtle changes in the external structure of OmCI, which mediates interaction with C5, when bound to LTB₄ or a non-physiological ligand such as palmitoleic acid (see Example 3).

### Example 7: OMCI alone and OMCI bound to human C5 exhibit the same binding kinetics to LTB₄

### Background

LTB₄ binding may be altered when OMCI is bound to C5. This could occur via steric hindrance, or via changes in enthalpy and/or conformation. The possibility was assessed by comparing the binding kinetics of radio labelled LTB₄ to OMCI and OMCI in complex with human C5 (hC5).

### Method:

Recombinant purified bacterial OMCI and hC5 (Calbiochem) at a 1:2 molar ratio were incubated in PBS at RT for 10 minutes to form the OMCI:hC5 complex. Formation of the complex was confirmed by native polyacrylamide gel shift (data not shown). Equal amounts OMCI:hC5 or OMCI alone were serially diluted in 75µl PBS before adding 75µl PBS containing ∼ 24000 c.p.m [5,6,8,9,11,12,14,15-³H(n)]-LTB₄ (Perkin Elmer, NEN Biotech, Lot 3589956; total activity 5µCi or 185kBq; specific activity 190 Ci/mmol). Following incubation (3h, RT), samples were centrifuged at 8000g for 2 minutes and the radioactivity remaining in solution measured on a Wallac 1217 Rackbeta liquid scintillation counter after transferring 20µl of the supernatant to 4ml Beckman Ready value scintillation cocktail. PBS only and serial dilutions of RaHBP2 and hC5 in PBS were used as negative controls.

### Results:

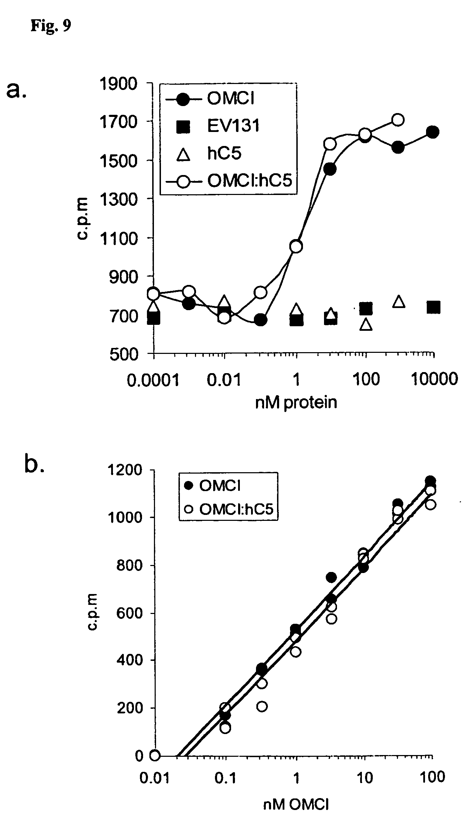

Fig. 9a shows that OMCI and OMCI:hC5 show saturable binding to ³H-LTB₄, whereas PBS (not shown), RaHBP2 and hC5 do not. The assay used here actually measures the ability of OMCI to bind LTB₄ and keep it in solution. No more than 20% of the labelled LTB₄ remained in solution in the negative control samples whereas more than50% remained in solution at the higher concentrations of OMCI (Fig. 9a). Association and dissociation constants cannot be accurately derived using this data, however comparison of the slope of the logarithmic regression functions for equivalent concentrations of OMCI and OMCI:hC5 indicate that the binding kinetics between LTB₄ and OMCI are not altered by binding to C5 (Fig. 9b). This data is in accord with the use of opposite faces of OMCI for C5 binding and the entry of LTB₄ to the lipocalin binding cavity.

### References

Aya, I. (2006) Blockade of leukotriene B4 signalling pathway directly inhibits cell proliferation and induces apoptosis colon cancer, Yokohama Medical Journal 57, 43-52.
Coligan, J. E. (1994). Complement. In "Current Protocols in Immunology". (J. E. Coligan, A. M. Kruisbeek, D. H. Marguiles, E. M. Shevach, and W. Strober, Eds.). Wiley Interscience.
Del Prete A., Shao W.H., Mitola S., Santoro G., Sozzani S., and Haribabu, B. (2007) Regulation of dendritic cell migration and adaptive immune response by leukotriene B4 receptors: a role for LTB4 in up-regulation of CCR7 expression and function. Blood, 109, 626-631.
Ford-Hutchinson, A. (1990). Leukotriene B4 in inflammation. Crit. Rev. Immunol. 10, 1-12.
Galbraith W.M., Hobson W.C., Giclas P.C., Schechter P.J. and Agrawal S. (1994) Complement activation and hemodynamic changes following intravenous administration of phosphorothioate oligonucleotides in the monkey. Antisense Res. Dev. 4(3):201-6.
Giclas, P.C. (1994). Classical and alternative pathway evaluation (sections 13.1 and 13.2). In Current Protocols in Immunology, Vol. 3, Complement. Editors: J.E. Coligan, A.M. Kruisbeek, D.H. Marguiles, E.M. Shevach and W. Strober. Series editor: R. Coico. John Wiley and Sons, Inc., USA.
Hao C.M. and Breyer M.D. (2007). Physiologic and pathophysiologic roles of lipid mediators in the kidney. Kidney International 71, 1105-1115.
Harrison, K. A., Murphy, R.C. (1995). Isoleukotrienes are biologically active free radical products of lipid peroxidation. J. Biol. Chem. 270, 17273-17276.
Hepburn, N.J., Williams, A.S., Nunn, M.A., Chamberlain-Banoub, J.C., Hamer, J., Morgan, B.P. and Harris, C.L. (2007) In vivo characterisation and therapeutic efficacy of CS-specific inhibitor from the soft tick Omithodoros moubata. J Biol Chem. 282, 8292-8299.
Hoover, H., Karnovasky, M., Austen, K., Corey, E. and Lewis, R. (1984). Leukotriene B4 action on endothelium mediates augmented neutrophil/endothelial adhesion. Proc. Nat. Acad. Sci. U.S.A. 81, 2191-2193.
Imig, J.D. (2000). Eicosanoid regulation of the renal vasculature. Am. J. Physiol. Renal Physiol. 279, F965-F981.
Klaas P.J.M. van Gisbergen, Marta Sanchez-Hennandez, Teunis B.H. Geijtenbeek, and Yvette van Kooyk (2005) Neutrophils mediate immune modulation of dendritic cells through glycosylation-dependent interactions between Mac-1 and DC-SIGN. J. Exp. Med. 201, 1281-1292.
Kim, N.D., Chou, R.C., Seung, E., Tager, A.M. and Luster, A.D. (2006). A unique requirement for the leukotriene B4 receptorBLT1 for neutrophil recruitment in inflammatory arthritis. J. Exp. Med. 203, 829-835.
Miyahara, N., Miyahara, S., Takeda, K., and Gelfand G.W. (2006). Role of the LTB4/BLT1 Pathway in Allergen-induced Airway Hyperresponsiveness and Inflammation. Allergol Int. 55, 91-7.
Nunn, M.A., Sharma, A., Paesen, G.C., Adamson, S., Lissina, O., Willis, A.C., & Nuttall, P.A. (2005) Complement inhibitor of C5 activation from the soft tick Ornithodoros moubata J. Immunol. 174, 2084-2091.
Samuelsson, B. (1983). Leukotrienes: mediators of immediate hypersensitivity reactions and inflammation. Science 220, 569-575.
Schwartz, G.K., Weitzman, A., O'Reilly, E., Brail, L., de Alwis, D.P., Cleverly, A., Barile-Thiem, B., Vinciguerra, V., and Budman D.R. (2005) Phase I and Pharmacokinetic Study of LY293111, an Orally Bioavailable LTB4 Receptor Antagonist, in Patients With Advanced Solid Tumors. Journal of Clinical Oncology, 23, 5365-5373.
Showell, H.J., Pettipher, E.R., Cheng, J.B., Breslow, R., Conklyn, M., Farrell, C.A, Hingorani, G.P., Salter, E.D., Hackman, B.C., Wimberly, D.J. et al (1995). The in vitro and in vivo pharmacologic activity of the potent and selective leukotriene B4 receptor antagonist CP-105696. J. Pharm. Exp. Ther. 273, 176-184.
Taube C., Miyahara N., Ott V., Swanson B., Takeda K., Loader J., Shultz L.D., Tager A.M., Luster A.D., Dakhama A., and Gelfand E.W. (2006) The leukotriene B4 receptor BLT1 is required for effector CD8+ T cell-mediated, mast cell-dependent airway hyperresponsiveness. J. Immunol. 176, 3157-3164.
Yamaoka, K A., Claesson, H.E., and Rosen, A. (1989) Leukotriene B4 enhances activation, proliferation, and differentiation of human B lymphocytes. J. Immunol. 143, 1996-2000.

## Claims

1. An OmCI polypeptide which has reduced or lacks LTB4 binding activity wherein said OmCI polypeptide comprises:
(a) an amino acid sequence of SEQ ID NO: 3 which has been modified to remove or reduce LTB4 binding activity;
(b) a variant amino acid sequence having at least 60% identity to the amino acid sequence of SEQ ID NO: 3 and which has been modified to remove or reduce LTB4 binding activity;
(c) a variant amino acid sequence of SEQ ID NO: 2 having at least 60% identity to the amino acid sequence between amino acid residues 19 to 168 of SEQ ID NO: 2 and which has been modified to remove or reduce LTB4 binding activity; or
(d) a fragment of the amino acid sequence of (a), (b) or (c) lacking LTB4 binding activity.

2. The polypeptide of claim 1 wherein one or more of the amino acid residues within the binding cavity of said OmCI polypeptide has been mutated.

3. The polypeptide of claim 2 wherein one or more of the amino acid residues to be mutated is selected from Phe36, Arg54, Leu57, Gly59, Val72, Met74, Phe76, Trp87, Phe89, Gln105, Arg107, His119, Asp121 and Trp133, wherein the numbering of amino acids is with reference to SEQ ID NO: 2.

4. The polypeptide of claim 2 or 3 wherein at least one amino acid mutation is selected from Phe36Trp and Gly59Trp.

5. A polypeptide according to any one of the preceding claims which lacks LTB4 binding activity.

6. A polynucleotide encoding the OmCI polypeptide of any one of the preceding claims.

7. A vector comprising a polynucleotide according to claim 6.

8. A host cell comprising a polynucleotide according to claim 6 or a vector according to claim 7.

9. A pharmaceutical composition comprising:
(a) an OmCI polypeptide according to any one of claims 1 to 5;
(b) a polynucleotide according to claim 6; or
(c) a vector according to claim 7;
and a pharmaceutically acceptable carrier.

## Patentansprüche

1. OmCI-Polypeptid mit reduzierter oder fehlender LTB4-Bindungsaktivität, wobei das OmCI-Polypeptid Folgendes umfasst:
(a) eine Aminosäuresequenz gemäß SEQ ID Nr. 3, die so modifiziert worden ist, dass die LTB4-Bindungsaktivität entfernt oder reduziert wird;
(b) eine Aminosäuresequenzvariante mit mindestens 60% Identität zu der Aminosäuresequenz gemäß SEQ ID Nr. 3, die so modifiziert worden ist, dass die LTB4-Bindungsaktivität entfernt oder reduziert wird;
(c) eine Aminosäuresequenzvariante gemäß SEQ ID Nr. 2 mit mindestens 60% Identität zu der Aminosäuresequenz zwischen den Aminosäureresten 19 bis 168 gemäß SEQ ID Nr. 2, die so modifiziert worden ist, dass die LTB4-Bindungsaktivität entfernt oder reduziert wird; oder
(d) ein Fragment der Aminosäuresequenz gemäß (a), (b) oder (c), dem LTB4-Bindungsaktivität fehlt.

2. Polypeptid nach Anspruch 1, wobei ein oder mehr der Aminosäurereste innerhalb der Bindungstasche des OmCI-Polypeptids mutiert worden ist.

3. Polypeptid nach Anspruch 2, wobei ein oder mehr der zu mutierenden Aminosäurereste aus Phe36, Arg54, Leu57, Gly59, Val72, Met74, Phe76, Trp87, Phe89, Gln105, Arg107, His119, Asp121 und Trp133 ausgewählt ist, wobei sich die Nummerierung der Aminosäuren auf SEQ ID Nr. 2 bezieht.

4. Polypeptid nach Anspruch 2 oder 3, wobei mindestens eine Aminosäuremutation aus Phe36Trp und Gly59Trp ausgewählt ist.

5. Polypeptid nach einem der vorhergehenden Ansprüche, dem LTB4-Bindungsaktivität fehlt.

6. Polypeptid, das für das OmCI-Polypeptid nach einem der vorhergehenden Ansprüche codiert.

7. Vektor, der ein Polynukleotid nach Anspruch 6 umfasst.

8. Wirtszelle, die ein Polynukleotid nach Anspruch 6 oder einen Vektor nach Anspruch 7 umfasst.

9. Pharmazeutische Zusammensetzung, die Folgendes umfasst:
(a) ein OmCI-Polypeptid nach einem der Ansprüche 1 bis 5;
(b) ein Polynukleotid nach Anspruch 6; oder
(c) einen Vektor nach Anspruch 7;
sowie einen pharmazeutisch unbedenklichen Träger.

## Revendications

1. Polypeptide OmCI qui possède une activité de liaison de LTB4 réduite ou absente, ledit polypeptide OmCI comprenant :
(a) une séquence d'acides aminés de SEQ ID NO: 3 qui a été modifiée pour éliminer ou réduire l'activité de liaison de LTB4 ;
(b) une séquence d'acides aminés variante ayant au moins 60 % d'identité avec la séquence d'acides aminés de SEQ ID NO: 3 et qui a été modifiée pour éliminer ou réduire l'activité de liaison de LTB4 ;
(c) une séquence d'acides aminés variante de SEQ ID NO: 2 ayant au moins 60 % d'identité avec la séquence d'acides aminés entre les résidus d'acide aminé 19 et 168 de SEQ ID NO: 2 et qui a été modifiée pour éliminer ou réduire l'activité de liaison de LTB4 ; ou
(d) un fragment de la séquence d'acides aminés de (a), (b) ou (c) ne présentant pas d'activité de liaison de LTB4.

2. Polypeptide de la revendication 1 dans lequel un ou plusieurs des résidus d'acide aminé dans la cavité de liaison dudit polypeptide OmCI ont été mutés.

3. Polypeptide de la revendication 2 dans lequel un ou plusieurs des résidus d'acide aminé à muter sont choisis parmi Phe36, Arg54, Leu57, Gly59, Val72, Met74, Phe76, Trp87, Phe89, Glnl05, Argl07, His119, Asp121 et Trp133, où la numérotation des acides aminés est en référence à SEQ ID NO: 2.

4. Polypeptide de la revendication 2 ou 3 dans lequel au moins une mutation d'acide aminé est choisie parmi Phe36Trp et Gly59Trp.

5. Polypeptide selon l'une quelconque des revendications précédentes qui ne présente pas d'activité de liaison de LTB4.

6. Polynucléotide codant pour le polypeptide OmCI de l'une quelconque des revendications précédentes.

7. Vecteur comprenant un polynucléotide selon la revendication 6.

8. Cellule hôte comprenant un polynucléotide selon la revendication 6 ou un vecteur selon la revendication 7.

9. Composition pharmaceutique comprenant :
(a) un polypeptide OmCI selon l'une quelconque des revendications 1 à 5 ;
(b) un polynucléotide selon la revendication 6 ; ou
(c) un vecteur selon la revendication 7 ;
et un véhicule pharmaceutiquement acceptable.


### SEQUENCE LISTING

<110> Natural Environment Research Council
<120> TREATMENT OF DISEASES AND CONDITIONS MEDIATED BY COMPLEMENT
<130> N.107303A
<150> PCT/GB2009/000311
   <151> 2009-02-05
<150> GB 0906779.4
   <151> 2009-04-20
<160> 11
<170> PatentIn version 3.4
<210> 1
   <211> 525
   <212> DNA
   <213> Ornithodoros moubata
<220>
   <221> CDS
   <222> (13)..(519)
<220>
   <221> sig_peptide
   <222> (13)..(66)
<400> 1
   taagagctca aa atg ctg gtt ttg gtg acc ctg att ttc tcc ttt tct gcg     51
                Met Leu Val Leu Val Thr Leu Ile Phe Ser Phe Ser Ala
                1               5                   10
   aac atc gca tat gct gac agc gaa agc gac tgc act gga agc gaa cct   99
   Asn Ile Ala Tyr Ala Asp Ser Glu Ser Asp Cys Thr Gly Ser Glu Pro
       15                  20                  25
   gtt gac gcc ttc caa gct ttc agt gag ggc aaa gag gca tat gtc ctg   147
   Val Asp Ala Phe Gln Ala Phe Ser Glu Gly Lys Glu Ala Tyr Val Leu
   30                  35                  40                  45
   gtg agg tcc acg gat ccc aaa gcg agg gac tgc ttg aaa gga gaa cca   195
   Val Arg Ser Thr Asp Pro Lys Ala Arg Asp Cys Leu Lys Gly Glu Pro
                   50                  55                  60
   gcc gga gaa aag cag gac aac acg ttg ccg gtg atg atg acg ttt aag   243
   Ala Gly Glu Lys Gln Asp Asn Thr Leu Pro Val Met Met Thr Phe Lys
               65                  70                  75
   aat ggc aca gac tgg gct tca acc gat tgg acg ttt act ttg gac ggc   291
   Asn Gly Thr Asp Trp Ala Ser Thr Asp Trp Thr Phe Thr Leu Asp Gly
           80                  85                  90
   gca aag gta acg gca acc ctt ggt aac cta acc caa aat agg gaa gtg   339
   Ala Lys Val Thr Ala Thr Leu Gly Asn Leu Thr Gln Asn Arg Glu Val
       95                  100                 105
   gtc tac gac tcg caa agt cat cac tgc cac gtt gac aag gtc gag aag   387
   Val Tyr Asp Ser Gln Ser His His Cys His Val Asp Lys Val Glu Lys
   110                 115                 120                 125
   gaa gtt cca gat tat gag atg tgg atg ctc gat gcg gga ggg ctt gaa   435
   Glu Val Pro Asp Tyr Glu Met Trp Met Leu Asp Ala Gly Gly Leu Glu
                   130                 135                 140
   gtg gaa gtc gag tgc tgc cgt caa aag ctt gaa gag ttg gcg tct ggc   483
   Val Glu Val Glu Cys Cys Arg Gln Lys Leu Glu Glu Leu Ala Ser Gly
               145                 150                 155
   agg aac caa atg tat ccc cat ctc aag gac tgc tag gcggcc            525
   Arg Asn Gln Met Tyr Pro His Leu Lys Asp Cys
           160                 165
<210> 2
   <211> 168
   <212> PRT
   <213> Ornithodoros moubata
<220>
   <223> Protein sequence including signal peptide at residues 1 to 18
<400> 2
   Met Leu Val Leu Val Thr Leu Ile Phe Ser Phe Ser Ala Asn Ile Ala
   1               5                   10                  15
   Tyr Ala Asp Ser Glu Ser Asp Cys Thr Gly Ser Glu Pro Val Asp Ala
               20                  25                  30
   Phe Gln Ala Phe Ser Glu Gly Lys Glu Ala Tyr Val Leu Val Arg Ser
           35                  40                  45
   Thr Asp Pro Lys Ala Arg Asp Cys Leu Lys Gly Glu Pro Ala Gly Glu
       50                  55                  60
   Lys Gln Asp Asn Thr Leu Pro Val Met Met Thr Phe Lys Asn Gly Thr
   65                  70                  75                  80
   Asp Trp Ala Ser Thr Asp Trp Thr Phe Thr Leu Asp Gly Ala Lys Val
                   85                  90                  95
   Thr Ala Thr Leu Gly Asn Leu Thr Gln Asn Arg Glu Val Val Tyr Asp
               100                 105                 110
   Ser Gln Ser His His Cys His Val Asp Lys Val Glu Lys Glu Val Pro
           115                 120                 125
   Asp Tyr Glu Met Trp Met Leu Asp Ala Gly Gly Leu Glu Val Glu Val
       130                 135                 140
   Glu Cys Cys Arg Gln Lys Leu Glu Glu Leu Ala Ser Gly Arg Asn Gln
   145                 150                 155                 160
   Met Tyr Pro His Leu Lys Asp Cys
                   165
<210> 3
   <211> 150
   <212> PRT
   <213> Ornithodoros moubata
<220>
   <223> Protein sequence lacking N terminal signal peptide
<400> 3
   Asp Ser Glu Ser Asp Cys Thr Gly Ser Glu Pro Val Asp Ala Phe Gln
   1               5                   10                  15
   Ala Phe Ser Glu Gly Lys Glu Ala Tyr Val Leu Val Arg Ser Thr Asp
               20                  25                  30
   Pro Lys Ala Arg Asp Cys Leu Lys Gly Glu Pro Ala Gly Glu Lys Gln
           35                  40                  45
   Asp Asn Thr Leu Pro Val Met Met Thr Phe Lys Asn Gly Thr Asp Trp
       50                  55                  60
   Ala Ser Thr Asp Trp Thr Phe Thr Leu Asp Gly Ala Lys Val Thr Ala
   65                  70                  75                  80
   Thr Leu Gly Asn Leu Thr Gln Asn Arg Glu Val Val Tyr Asp Ser Gln
                   85                  90                  95
   Ser His His Cys His Val Asp Lys Val Glu Lys Glu Val Pro Asp Tyr
               100                 105                 110
   Glu Met Trp Met Leu Asp Ala Gly Gly Leu Glu Val Glu Val Glu Cys
           115                 120                 125
   Cys Arg Gln Lys Leu Glu Glu Leu Ala Ser Gly Arg Asn Gln Met Tyr
       130                 135                 140
   Pro His Leu Lys Asp Cys
   145                 150
<210> 4
   <211> 525
   <212> DNA
   <213> Artificial sequence
<220>
   <221> CDS
   <222> (13)..(519)
<220>
   <221> sig_peptide
   <222> (13)..(66)
<220>
   <223> Sequence of SEQ ID NO:1 altered by site directed mutagenesis to
   prevent hyperglycosylation of encoded peptide. Codons at positions 244-246 and 316-318 are replaced with caa.
<400> 4
   taagagctca aa atg ctg gtt ttg gtg acc ctg att ttc tcc ttt tct gcg 51
                Met Leu Val Leu Val Thr Leu Ile Phe Ser Phe Ser Ala
                1               5                   10
   aac atc gca tat gct gac agc gaa agc gac tgc act gga agc gaa cct   99
   Asn Ile Ala Tyr Ala Asp Ser Glu Ser Asp Cys Thr Gly Ser Glu Pro
       15                  20                  25
   gtt gac gcc ttc caa gct ttc agt gag ggc aaa gag gca tat gtc ctg  147
   Val Asp Ala Phe Gln Ala Phe Ser Glu Gly Lys Glu Ala Tyr Val Leu
   30                  35                  40                  45
   gtg agg tcc acg gat ccc aaa gcg agg gac tgc ttg aaa gga gaa cca  195
   Val Arg Ser Thr Asp Pro Lys Ala Arg Asp Cys Leu Lys Gly Glu Pro
                   50                  55                  60
   gcc gga gaa aag cag gac aac acg ttg ccg gtg atg atg acg ttt aag  243
   Ala Gly Glu Lys Gln Asp Asn Thr Leu Pro Val Met Met Thr Phe Lys
               65                  70                  75
   caa ggc aca gac tgg gct tca acc gat tgg acg ttt act ttg gac ggc  291
   Gln Gly Thr Asp Trp Ala Ser Thr Asp Trp Thr Phe Thr Leu Asp Gly
           80                  85                  90
   gca aag gta acg gca acc ctt ggt caa cta acc caa aat agg gaa gtg  339
   Ala Lys Val Thr Ala Thr Leu Gly Gln Leu Thr Gln Asn Arg Glu Val
       95                  100                 105
   gtc tac gac tcg caa agt cat cac tgc cac gtt gac aag gtc gag aag  387
   Val Tyr Asp Ser Gln Ser His His Cys His Val Asp Lys Val Glu Lys
   110                 115                 120                 125
   gaa gtt cca gat tat gag atg tgg atg ctc gat gcg gga ggg ctt gaa  435
   Glu Val Pro Asp Tyr Glu Met Trp Met Leu Asp Ala Gly Gly Leu Glu
                   130                 135                 140
   gtg gaa gtc gag tgc tgc cgt caa aag ctt gaa gag ttg gcg tct ggc  483
   Val Glu Val Glu Cys Cys Arg Gln Lys Leu Glu Glu Leu Ala Ser Gly
               145                 150                 155
   agg aac caa atg tat ccc cat ctc aag gac tgc tag gcggcc           525
   Arg Asn Gln Met Tyr Pro His Leu Lys Asp Cys
           160                 165
<210> 5
   <211> 168
   <212> PRT
   <213> Artificial sequence
<220>
   <223> Peptide Sequence encoded by SEQ ID NO:4. Site directed mutagenesis of SEQ ID NO: 4 results in Gln in place of Asn at positions 78 and 102 as compared with SEQ ID NO:2 to prevent hyperglycosylation.
<400> 5
   Met Leu Val Leu Val Thr Leu Ile Phe Ser Phe Ser Ala Asn Ile Ala
   1               5                   10                  15
   Tyr Ala Asp Ser Glu Ser Asp Cys Thr Gly Ser Glu Pro Val Asp Ala
               20                  25                  30
   Phe Gln Ala Phe Ser Glu Gly Lys Glu Ala Tyr Val Leu Val Arg Ser
           35                  40                  45
   Thr Asp Pro Lys Ala Arg Asp Cys Leu Lys Gly Glu Pro Ala Gly Glu
       50                  55                  60
   Lys Gln Asp Asn Thr Leu Pro Val Met Met Thr Phe Lys Gln Gly Thr
   65                  70                  75                  80
   Asp Trp Ala Ser Thr Asp Trp Thr Phe Thr Leu Asp Gly Ala Lys Val
                   85                  90                  95
   Thr Ala Thr Leu Gly Gln Leu Thr Gln Asn Arg Glu Val Val Tyr Asp
               100                 105                 110
   Ser Gln Ser His His Cys His Val Asp Lys Val Glu Lys Glu Val Pro
           115                 120                 125
   Asp Tyr Glu Met Trp Met Leu Asp Ala Gly Gly Leu Glu Val Glu Val
       130                 135                 140
   Glu Cys Cys Arg Gln Lys Leu Glu Glu Leu Ala Ser Gly Arg Asn Gln
   145                 150                 155                 160
   Met Tyr Pro His Leu Lys Asp Cys
                   165
<210> 6
   <211> 525
   <212> DNA
   <213> Artifical sequence
<220>
   <221> CDS
   <222> (13)..(519)
<220>
   <221> sig_peptide
   <222> (13)..(66)
<220>
   <223> Ornithodoros moubata mutant OmCI-F36W: sequence of SEQ ID NO:1 altered by site directed mutagenesis to change phenylalanine (F) 36 to tryptophan (W) to prevent fatty acid binding Codons at positions 118-120 are replaced with tgg
<400> 6
   taagagctca aa atg ctg gtt ttg gtg acc ctg att ttc tcc ttt tct gcg  51
                   Met Leu Val Leu Val Thr Leu Ile Phe Ser Phe Ser Ala
                   1               5                   10
   aac atc gca tat gct gac agc gaa agc gac tgc act gga agc gaa cct   99
   Asn Ile Ala Tyr Ala Asp Ser Glu Ser Asp Cys Thr Gly Ser Glu Pro
       15                  20                  25
   gtt gac gcc ttc caa gct tgg agt gag ggc aaa gag gca tat gtc ctg   147
   Val Asp Ala Phe Gln Ala Trp Ser Glu Gly Lys Glu Ala Tyr Val Leu
   30                  35                  40                  45
   gtg agg tcc acg gat ccc aaa gcg agg gac tgc ttg aaa gga gaa cca   195
   Val Arg Ser Thr Asp Pro Lys Ala Arg Asp Cys Leu Lys Gly Glu Pro
                   50                  55                  60
   gcc gga gaa aag cag gac aac acg ttg ccg gtg atg atg acg ttt aag   243
   Ala Gly Glu Lys Gln Asp Asn Thr Leu Pro Val Met Met Thr Phe Lys
               65                  70                  75
   aat ggc aca gac tgg gct tca acc gat tgg acg ttt act ttg gac ggc   291
   Asn Gly Thr Asp Trp Ala Ser Thr Asp Trp Thr Phe Thr Leu Asp Gly
           80                  85                  90
   gca aag gta acg gca acc ctt ggt aac cta acc caa aat agg gaa gtg   339
   Ala Lys Val Thr Ala Thr Leu Gly Asn Leu Thr Gln Asn Arg Glu Val
       95                  100                 105
   gtc tac gac tcg caa agt cat cac tgc cac gtt gac aag gtc gag aag   387
   Val Tyr Asp Ser Gln Ser His His Cys His Val Asp Lys Val Glu Lys
   110                 115                 120                 125
   gaa gtt cca gat tat gag atg tgg atg ctc gat gcg gga ggg ctt gaa   435
   Glu Val Pro Asp Tyr Glu Met Trp Met Leu Asp Ala Gly Gly Leu Glu
                   130                 135                 140
   gtg gaa gtc gag tgc tgc cgt caa aag ctt gaa gag ttg gcg tct ggc   483
   Val Glu Val Glu Cys Cys Arg Gln Lys Leu Glu Glu Leu Ala Ser Gly
               145                 150                 155
   agg aac caa atg tat ccc cat ctc aag gac tgc tag gcggcc            525
   Arg Asn Gln Met Tyr Pro His Leu Lys Asp Cys
           160                 165
<210> 7
   <211> 168
   <212> PRT
   <213> Artificial sequence
<220>
   <223> Ornithodoros moubata mutant OmCI-F36W Protein sequence including signal peptide at residues 1 to 18
<400> 7
   Met Leu Val Leu Val Thr Leu Ile Phe Ser Phe Ser Ala Asn Ile Ala
   1               5                   10                  15
   Tyr Ala Asp Ser Glu Ser Asp Cys Thr Gly Ser Glu Pro Val Asp Ala
   20 25 30
   Phe Gln Ala Trp Ser Glu Gly Lys Glu Ala Tyr Val Leu Val Arg Ser
   35 40 45
   Thr Asp Pro Lys Ala Arg Asp Cys Leu Lys Gly Glu Pro Ala Gly Glu
   50 55 60
   Lys Gln Asp Asn Thr Leu Pro Val Met Met Thr Phe Lys Asn Gly Thr
   65 70 75 80
   Asp Trp Ala Ser Thr Asp Trp Thr Phe Thr Leu Asp Gly Ala Lys Val
   85 90 95
   Thr Ala Thr Leu Gly Asn Leu Thr Gln Asn Arg Glu Val Val Tyr Asp
   100 105 110
   Ser Gln Ser His His Cys His Val Asp Lys Val Glu Lys Glu Val Pro
   115 120 125
   Asp Tyr Glu Met Trp Met Leu Asp Ala Gly Gly Leu Glu Val Glu Val
   130 135 140
   Glu Cys Cys Arg Gln Lys Leu Glu Glu Leu Ala Ser Gly Arg Asn Gln
   145 150 155 160
   Met Tyr Pro His Leu Lys Asp Cys
   165
<210> 8
   <211> 150
   <212> PRT
   <213> Artificial sequence
<220>
   <223> Ornithodoros moubata OMCI mutant OmCI-F36W Protein sequence lacking N terminal signal peptide
<400> 8
   Asp Ser Glu Ser Asp Cys Thr Gly Ser Glu Pro Val Asp Ala Phe Gln
   1 5 10 15
   Ala Trp Ser Glu Gly Lys Glu Ala Tyr Val Leu Val Arg Ser Thr Asp
   20 25 30
   Pro Lys Ala Arg Asp Cys Leu Lys Gly Glu Pro Ala Gly Glu Lys Gln
               35                  40                  45
   Asp Asn Thr Leu Pro Val Met Met Thr Phe Lys Asn Gly Thr Asp Trp
       50                  55                  60
   Ala Ser Thr Asp Trp Thr Phe Thr Leu Asp Gly Ala Lys Val Thr Ala
   65                  70                  75                  80
   Thr Leu Gly Asn Leu Thr Gln Asn Arg Glu Val Val Tyr Asp Ser Gln
                   85                  90                  95
   Ser His His Cys His Val Asp Lys Val Glu Lys Glu Val Pro Asp Tyr
               100                 105                 110
   Glu Met Trp Met Leu Asp Ala Gly Gly Leu Glu Val Glu Val Glu Cys
           115                 120                 125
   Cys Arg Gln Lys Leu Glu Glu Leu Ala Ser Gly Arg Asn Gln Met Tyr
       130                 135                 140
   Pro His Leu Lys Asp Cys
   145                 150
<210> 9
   <211> 525
   <212> DNA
   <213> Artificial sequence
<220>
   <221> CDS
   <222> (13)..(519)
<220>
   <221> sig_peptide
   <222> (13)..(66)
<220>
   <223> Ornithodoros moubata mutant OmCI-G59W: sequence of SEQ ID NO:1 altered by site directed mutagenesis to change glycine (F) 59 to tryptopha (W) to prevent fatty acid binding Codons at positions 187-189 are replaced with tgg.
<400> 9
   taagagctca aa atg ctg gtt ttg gtg acc ctg att ttc tcc ttt tct gcg   51
                 Met Leu Val Leu Val Thr Leu Ile Phe Ser Phe Ser Ala
                 1               5                   10
   aac atc gca tat gct gac agc gaa agc gac tgc act gga agc gaa cct   99
   Asn Ile Ala Tyr Ala Asp Ser Glu Ser Asp Cys Thr Gly Ser Glu Pro
       15                  20                  25
   gtt gac gcc ttc caa gct ttc agt gag ggc aaa gag gca tat gtc ctg  147
   Val Asp Ala Phe Gln Ala Phe Ser Glu Gly Lys Glu Ala Tyr Val Leu
   30                  35                  40                  45
   gtg agg tcc acg gat ccc aaa gcg agg gac tgc ttg aaa tgg gaa cca  195
   Val Arg Ser Thr Asp Pro Lys Ala Arg Asp Cys Leu Lys Trp Glu Pro
                   50                  55                  60
   gcc gga gaa aag cag gac aac acg ttg ccg gtg atg atg acg ttt aag  243
   Ala Gly Glu Lys Gln Asp Asn Thr Leu Pro Val Met Met Thr Phe Lys
               65                  70                  75
   aat ggc aca gac tgg gct tca acc gat tgg acg ttt act ttg gac ggc  291
   Asn Gly Thr Asp Trp Ala Ser Thr Asp Trp Thr Phe Thr Leu Asp Gly
           80                  85                  90
   gca aag gta acg gca acc ctt ggt aac cta acc caa aat agg gaa gtg  339
   Ala Lys Val Thr Ala Thr Leu Gly Asn Leu Thr Gln Asn Arg Glu Val
       95                  100                 105
   gtc tac gac tcg caa agt cat cac tgc cac gtt gac aag gtc gag aag  387
   Val Tyr Asp Ser Gln Ser His His Cys His Val Asp Lys Val Glu Lys
   110                 115                 120                 125
   gaa gtt cca gat tat gag atg tgg atg ctc gat gcg gga ggg ctt gaa  435
   Glu Val Pro Asp Tyr Glu Met Trp Met Leu Asp Ala Gly Gly Leu Glu
                   130                 135                 140
   gtg gaa gtc gag tgc tgc cgt caa aag ctt gaa gag ttg gcg tct ggc  483
   Val Glu Val Glu Cys Cys Arg Gln Lys Leu Glu Glu Leu Ala Ser Gly
               145                 150                 155
   agg aac caa atg tat ccc cat ctc aag gac tgc tag gcggcc           525
   Arg Asn Gln Met Tyr Pro His Leu Lys Asp Cys
           160                 165
<210> 10
   <211> 168
   <212> PRT
   <213> Artificial sequence
<220>
   <223> Ornithodoros moubata OMCI mutant OmCI-G59W Protein sequence including signal peptide at residues 1 to 18
<210> 11
   <211> 150
   <212> PRT
   <213> Artificial sequence
<220>
   <223> Ornithodoros moubata OMCI mutant OmCI-G59W Protein sequence lacking N terminal signal peptide
<400> 11
   Asp Ser Glu Ser Asp Cys Thr Gly Ser Glu Pro Val Asp Ala Phe Gln
   1               5                   10                  15
   Ala Phe Ser Glu Gly Lys Glu Ala Tyr Val Leu Val Arg Ser Thr Asp
               20                  25                  30
   Pro Lys Ala Arg Asp Cys Leu Lys Trp Glu Pro Ala Gly Glu Lys Gln
           35                  40                  45
   Asp Asn Thr Leu Pro Val Met Met Thr Phe Lys Asn Gly Thr Asp Trp
       50                  55                  60
   Ala Ser Thr Asp Trp Thr Phe Thr Leu Asp Gly Ala Lys Val Thr Ala
   65                  70                  75                  80
   Thr Leu Gly Asn Leu Thr Gln Asn Arg Glu Val Val Tyr Asp Ser Gln
                   85                  90                  95
   Ser His His Cys His Val Asp Lys Val Glu Lys Glu Val Pro Asp Tyr
               100                 105                 110
   Glu Met Trp Met Leu Asp Ala Gly Gly Leu Glu Val Glu Val Glu Cys
           115                 120                 125
   Cys Arg Gln Lys Leu Glu Glu Leu Ala Ser Gly Arg Asn Gln Met Tyr
       130                 135                 140
   Pro His Leu Lys Asp Cys
   145                 150